# EUROPÄISCHE PATENTSCHRIFT

(11) **EP 2 583 557 B1**
(45) Veröffentlichungstag und Bekanntmachung des Hinweises auf die Patenterteilung: **13.01.2016**
(21) Anmeldenummer: 12191543.3
(22) Anmeldetag: 04.07.2009
(51) Int. Cl.: A01N 43/824, A01N 43/78, A01N 43/56, A01N 43/836, A01N 43/88, A01N 47/38, A01P 7/04, C07D 417/14, C07D 401/14

(54) **Heterocyclische Verbindungen als Schädlingsbekämpfungsmittel**
Heterocyclic compounds as pest controllers
Liaisons hétérocycliques en tant que moyen de lutte contre les parasites

(30) Priorität: 17.07.2008 EP 08012898
(43) Veröffentlichungstag der Anmeldung: 24.04.2013
(62) Teilanmeldung aus: 09776952.5
(73) Patentinhaber: Bayer CropScience AG, 40789 Monheim (DE)
(72) Erfinder: Bretschneider, Thomas, 5397 Lohmar (DE); Füßlein Martin,, 40225 Düsseldorf (DE); Hense Achim, 51379 Leverkusen (DE); Kluth Joachim,, 40764 Langengelod (DE); Franken, Eva-Maria, 51381 Leverkusen (DE); Görgens, Ulrich, 40882 Ratingen, (DE); Schwarz, Hans-Georg, 46282 Dorsten (DE); Köhler, Adeline, 42105 Wuppertal (DE); Malsam Olga,, 51503 Rösrath (DE); Voerste Arnd,, 50674 Köln (DE); Becker Angela,, 40629 Düsseldorf (DE)

(56) Entgegenhaltungen:
- WO-A1-97/30050
- CH-A- 409 511

## Beschreibung

Die vorliegende Anmeldung betrifft die Verwendung zum Teil bekannter heterocyclischer Verbindungen zur Bekämpfung von tierischen Schädlingen, zu denen Arthropoden und insbesondere Insekten zählen, ferner neue heterocyclische Verbindungen und Verfahren zu deren Herstellung.

Bestimmte Thiazolylverbindungen sind bereits bekannt geworden, eine Verwendung zur Bekämpfung tierischer Schädlinge wurde jedoch nicht beschrieben (vgl. WO 2003/015776).

Ferner sind bekannt geworden Thiadiazolverbindungen. CH 411 906 und EP 0 288 432 A1 beschreiben die Verwendung solcher Verbindungen als optische Aufheller. In CH 409 511 werden Thiadiazole offenbart, die zur Bekämpfung von Nematoden geeignet sind. DE 3641184 beschreibt bestimmte phenylsubstituierte Thiadiazole zur Bekämpfung von Schädlingen.

In WO 1998/056785 und WO 1996/032938 werden Pyrazolverbindungen offenbart, für die pharmazeutische Verwendungen angegeben werden.

In WO 97/30050 A1 werden Heterocyclyl-Ergolin-Derivate als 5-HT1A Rezeptor Liganden beschrieben.

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch zum Teil neue Verbindungen der Formel (I), 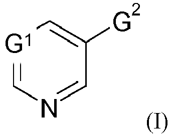 worin
(Ib)
- G¹: für N oder C-Halogen steht,
- G²: für 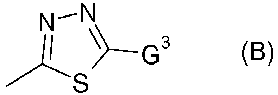
steht, worin
- G³: für gegebenenfalls durch Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: gegebenenfalls durch Halogen, Nitro, Amino, Alkylamino, Dialkylamino, Alkyl, Halogenalkyl, Cycloalkylalkyl, Alkoxy, Halogenalkoxy, Alkylthio, Alkoxyalkyl, Bis(alkoxy)alkyl, Alkoxycarbonyl, alpha-Hydroxyimino-alkoxycarbonylmethyl, alpha-Alkoxyimino-alkoxycarbonylmethyl, C(X)NR²R³, (worin X für Sauerstoff oder Schwefel steht, R² für Wasserstoff oder Alkyl steht und R³ für Alkyl, Halogenalkyl, Alkoxy, Cyanoalkyl, Alkinyl, Cycloalkyl, Cycloalkylalkyl, Alkoxyalkyl, Alkylthioalkyl oder Arylalkyl steht oder R² und R³ gemeinsam mit dem Stickstoffatom, an das sie gebunden sind, einen Ring bilden), Alkylthio, Alkylsulfinyl, Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch Alkyl oder Halogenalkyl), Phenyl (welches selbst wiederum substituiert sein kann durch Halogen, Cyano, Nitro, Alkyl oder Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl), die Heteroarylalkylreste Triazolylalkyl, Pyridylalkyl, Pyrimidylalkyl oder Oxadiazolylalkyl (welche selbst wiederum substituiert sein können durch Alkyl) substituiertes Hetaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, insbesondere Pyridyl, Pyrimidyl, Imidazolyl, Pyrazolyl, Triazinyl, Thiazolyl, Thiadiazolyl, Oxadiazolyl und Oxazolyl oder
- für: gegebenenfalls durch Halogen, Cyano, Alkyl, Halogenalkyl, Alkoxy, Halogenalkoxy, Piperidinonyl, Pyrrolidinonyl, Dioxolanyl oder Dihydrodioxazinyl substituiertes Phenyl steht,
sowie Salze, Metallkomplexe und N-Oxide der Verbindungen der Formel (I),
die zur Bekämpfung von Schädlingen verwendet werden können.

Es wurde gefunden, dass die Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die bekannten Verbindungen der Formel (I) sind nach den Herstellverfahren erhältlich, die in den oben genannten Publikationen beschrieben sind.

Bevorzugte Substituenten bzw. Bereiche der in den oben erwähnten Verbindungen (Ib) aufgeführten Reste werden im Folgenden erläutert.

Verwendet werden bevorzugt Verbindungen der Formel (I), 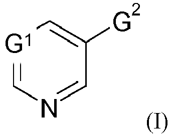 worin
(Ib)
- G¹: für N oder C-Halogen steht,
- G²: für 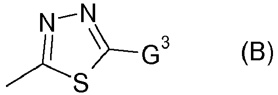
steht, worin
- G³: jeweils für gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₁-C₆-Alkylcarbonyl, C₁-C₆-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: jeweils gegebenenfalls durch Halogen, Nitro, Amino, C₁-C₆-Alkylamino, Di-(C₁-C₆-alkyl)amino, C₁-C₆-Alkyl, C₁-C₆-Halogenalkyl, C₃-C₆-Cycloalkyl-C₁-C₆-alkyl, C₁-C₆-Alkoxy, C₁-C₆-Halogenalkoxy, C₁-C₆-Alkylthio, C₁-C₆-Alkoxy-C₁-C₆-alkyl, Bis(C₁-C₆-alkoxy)-C₁-C₆-alkyl, C₁-C₆-Alkoxycarbonyl, alpha-Hydroxyimino-C₁-C₆-alkoxycarbonylmethyl, alpha- C₁-C₆-Alkoxyimino-C₁-C₆-alkoxycarbonylmethyl, C(X)NR²R³, (worin X für Sauerstoff oder Schwefel steht, R² für Wasserstoff oder C₁-C₆-Alkyl steht und R³ für C₁-C₆-Alkyl, C₁-C₆-Halogenalkyl, C₁-C₆-Alkoxy, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkylthio-C₁-C₆-alkyl oder Phenyl-C₁-C₆-alkyl steht), C₁-C₆-Alkylsulfinyl, C₁-C₆-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₆-Alkyl oder C₁-C₆-Halogenalkyl), Phenyl (welches selbst wiederum substituiert sein kann durch Halogen, Cyano, Nitro, C₁-C₆-Alkyl oder C₁-C₆-Halogenalkyl), die Heteroarylreste Pyrrolyl Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl), die Heteroarylalkylreste Triazolyl-C₁-C₆-alkyl, Pyridyl-C₁-C₆-alkyl, Pyrimidyl-C₁-C₆-alkyl oder Oxadiazolyl-C_{I}-C₆-alkyl (welche selbst wiederum substituiert sein können durch C_{I}-C₆-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyridyl, Pyrimidyl, Imidazolyl, Pyrazolyl, Triazinyl, Thiazolyl, Thiadiazolyl, Oxadiazolyl oder Oxazolyl) oder
- für: gegebenenfalls durch Halogen, Cyano, C₁-C₆-Alkyl, C₁-C₆-Halogenalkyl, C₁-C₆-Alkoxy, C₁-C₆-Halogenalkoxy, Dioxolanyl, Piperidinonyl, Pyrrolidinonyl oder Dihydrodioxazinyl substituiertes Phenyl steht,
sowie Salze und N-Oxide der Verbindungen der Formel (I).

Verwendet werden besonders bevorzugt Verbindungen der Formel (I), 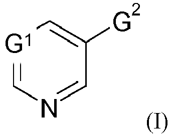 worin
(Ib)
- G¹: für N oder C-Halogen steht,
- Dz ₂: für 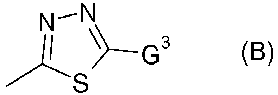
steht, worin
- er: jeweils für gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₁-C₄-Alkylcarbonyl, C₁-C₄-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: gegebenenfalls durch Halogen, Nitro, Amino, C₁-C₄-Alkylamino, Di-(C₁-C₄-alkyl)amino, C₁-C₄-Alkyl, C₁-C₄-Halogenalkyl, C₃-C₆-Cycloalkyl-C₁-C₄-alkyl, C₁-C₄-Alkoxy, C₁-C₄-Halogenalkoxy, C₁-C₄-Alkylthio, C₁-C₄-Alkoxy-C₁-C₄-alkyl, Bis(C₁-C₄-alkoxy)-C₁-C₄-alkyl, C₁-C₄-Alkoxycarbonyl, alpha-Hydroxyimino-C₁-C₄-alkoxycarbonylmethyl, alpha- C₁-C₄-Alkoxyimino-C₁-C₄-alkoxycarbonylmethyl, C(X)NR²R³, (worin X für Sauerstoff oder Schwefel steht, R² für Wasserstoff oder C₁-C₄-Alkyl steht und R³ für C₁-C₄-Alkyl, C₁-C₄-Halogenalkyl, C₁-C₆-Alkoxy, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht), C₁-C₄-Alkylsulfinyl, C₁-C₄-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₄-Alkyl oder C₁-C₄-Halogenalkyl), Phenyl (welches selbst wiederum substituiert sein kann durch Halogen, Cyano, Nitro, C₁-C₄-Alkyl oder C₁-C₄-Halogenalkyl), die Heteroarylreste Pyrrolyl Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl), die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch C₁-C₄-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benziso-thienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyridyl, Pyrimidyl, Imidazolyl, Pyrazolyl, Triazinyl, Thiazolyl, Thiadiazolyl, Oxadiazolyl oder Oxazolyl) oder
- für: gegebenenfalls durch Halogen, Cyano, C₁-C₄-Alkyl, C₁-C₄-Halogenalkyl, C₁-C₄-Alkoxy, C₁-C₄-Halogenalkoxy, Dioxolanyl, Piperidinonyl, Pyrrolidinonyl oder Dihydrodioxazinyl substituiertes Phenyl steht,
sowie Salze und N-Oxide der Verbindungen der Formel (I).

Verwendet werden ganz besonders bevorzugt Verbindungen der Formel (I), 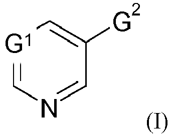 worin
(Ib)
- G¹: für N oder C-Halogen (insbesondere CF) steht,
- G²: für 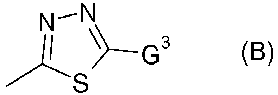
steht, worin
- G³: für gegebenenfalls durch Pyrrolyl oder Pyrimidinyl substituiertes Pyridyl steht,
sowie Salze und N-Oxide der Verbindungen der Formel (I).

Durch Halogen (auch "Halo" abgekürzt) substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom, hervorgehoben für Fluor und Chlor.

Der Rest "Pyrimidyl" wird auch als "Pyrimidinyl" bezeichnet.

Bevorzugt, besonders bevorzugt oder ganz besonders bevorzugt verwendet werden Verbindungen, welche jeweils die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

In einer weiteren hervorgehobenen Gruppe von erfindungsgemäß zu verwendenden Verbindungen (Ib) steht G¹ für N.

In einer weiteren hervorgehobenen Gruppe von erfindungsgemäß zu verwendenden Verbindungen (Ib) steht G¹ für C-Halogen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Erfindung betrifft auch neue Verbindungen der Formel (IB-1) 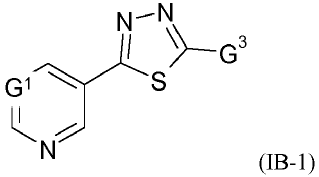 worin
- G¹: für N oder C-Halogen steht,
- G³: jeweils für gegebenenfalls durch Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: gegebenenfalls durch Halogen, Nitro, Amino, Alkylamino, Dialkylamino, Alkyl, Halogenalkyl, Cycloalkylalkyl, Alkoxy, Halogenalkoxy, Alkylthio, Alkoxyalkyl, Bis(alkoxy)alkyl, Alkoxycarbonyl, alpha-Hydroxyimino-alkoxycarbonylmethyl, alpha-Alkoxyimino-alkoxycarbonylmethyl, C(X)NR²R³, (worin X für Sauerstoff oder Schwefel steht, R² für Wasserstoff oder Alkyl steht und R³ für Alkyl, Halogenalkyl, Alkoxy, Cyanoalkyl, Alkinyl, Cycloalkyl, Cycloalkylalkyl, Alkoxyalkyl, Alkylthioalkyl oder Arylalkyl steht), Alkylthio, Alkylsulfinyl, Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch Alkyl oder Halogenalkyl), Phenyl (welches selbst wiederum substituiert sein kann durch Halogen, Cyano, Nitro, Alkyl oder Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl), die Heteroarylalkylreste Triazolylalkyl, Pyridylalkyl, Pyrimidylalkyl oder Oxadiazolylalkyl (welche selbst wiederum substituiert sein können durch Alkyl) substituiertes Hetaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, insbesondere Pyridyl, Pyrimidyl, Imidazolyl, Pyrazolyl, Triazinyl, Thiazolyl, Thiadiazolyl, Oxadiazolyl und Oxazolyl oder
- für: gegebenenfalls durch Halogen, Cyano, Alkyl, Halogenalkyl, Alkoxy, Halogenalkoxy Dioxolanyl, Piperidinonyl, Pyrrolidinonyl oder Dihydrodioxazinyl substituiertes Phenyl steht.

Bevorzugt sind neue Verbindungen der Formel (IB-1), worin
- G¹: für N oder C-Halogen steht,
- G³: jeweils für gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₁-C₆-Alkylcarbonyl, C₁-C₆-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: jeweils gegebenenfalls durch Halogen, Nitro, Amino, C₁-C₆-Alkylamino, Di-(C₁-C₆-alkyl)amino, C₁-C₆-Alkyl, C₁-C₆-Halogenalkyl, C₃-C₆-Cycloalkyl-C₁-C₆-alkyl, C₁-C₆-Alkoxy, C₁-C₆-Halogenalkoxy, C₁-C₆-Alkylthio, C₁-C₆-Alkoxy-C₁-C₆-alkyl, Bis(C₁-C₆-alkoxy)-C₁-C₆-alkyl, C₁-C₆-Alkoxycarbonyl, alpha-Hydroxyimino-C₁-C₆-alkoxycarbonylmethyl, alpha- C₁-C₆-Alkoxyimino-C₁-C₆-alkoxycarbonylmethyl, C(X)NR²R³, (worin X für Sauerstoff oder Schwefel steht, R² für Wasserstoff oder C₁-C₆-Alkyl steht und R³ für C₁-C₆-Alkyl, C₁-C₆-Halogenalkyl, C₁-C₆-Alkoxy, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkylthio-C₁-C₆-alkyl oder Phenyl-C₁-C₆-alkyl steht), C₁-C₆-Alkylsulfinyl, C₁-C₆-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₆-Alkyl oder C₁-C₆-Halogenalkyl), Phenyl (welches selbst wiederum substituiert sein kann durch Halogen, Cyano, Nitro, C₁-C₆-Alkyl oder C₁-C₆-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl), die Heteroarylalkylreste Triazolyl-C₁-C₆-alkyl, Pyridyl-C₁-C₆-alkyl, Pyrimidyl-C₁-C₆-alkyl oder Oxadiazolyl-C₁-C₆-alkyl (welche selbst wiederum substituiert sein können durch C₁-C₆-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyridyl, Pyrimidyl, Imidazolyl, Pyrazolyl, Triazinyl, Thiazolyl, Thiadiazolyl, Oxadiazolyl oder Oxazolyl) oder
- für: gegebenenfalls durch Halogen, Cyano, C₁-C₆-Alkyl, C₁-C₆-Halogenalkyl, C₁-C₆-Alkoxy, C₁-C₆-Halogenalkoxy, Dioxolanyl, Piperidinonyl, Pyrrolidinonyl oder Dihydrodioxazinyl substituiertes Phenyl steht.

Besonders bevorzugt sind neue Verbindungen der Formel (IB-1), worin
- G¹: für N oder C-Halogen steht,
- G³: jeweils für gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₁-C₄-Alkylcarbonyl, C₁-C₄-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
- für: jeweils gegebenenfalls durch Halogen, Nitro, Amino, C₁-C₄-Alkylamino, Di-(C₁-C₄-alkyl)amino, C₁-C₄-Alkyl, C₁-C₄-Halogenalkyl, C₃-C₆-Cycloalkyl-C₁-C₄-alkyl, C₁-C₄-Alkoxy, C₁-C₄-Halogenalkoxy, C₁-C₄-Alkylthio, C₁-C₄-Alkoxy-C₁-C₄-alkyl, Bis(C₁-C₄-alkoxy)-C₁-C₄-alkyl, C₁-C₄-Alkoxycarbonyl, alpha-Hydroxyimino-C₁-C₄-alkoxycarbonylmethyl, alpha- C₁-C₄-Alkoxyimino-C₁-C₄-alkoxycarbonylmethyl, C(X)NR²R³, (worin X für Sauerstoff oder Schwefel steht, R² für Wasserstoff oder C₁-C₄-Alkyl steht und R³ für C₁-C₄-Alkyl, C₁-C₄-Halogenalkyl, C₁-C₄-Alkoxy, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht), C₁-C₄-Alkylsulfinyl, C₁-C₄-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₄-Alkyl oder C₁-C₄-Halogenalkyl), Phenyl (welches selbst wiederum substituiert sein kann durch Halogen, Cyano, Nitro, C₁-C₄-Alkyl oder C₁-C₄-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl), die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch C₁-C₄-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyridyl, Pyrimidyl, Imidazolyl, Pyrazolyl, Triazinyl, Thiazolyl, Thiadiazolyl, Oxadiazolyl oder Oxazolyl) oder
- für: gegebenenfalls durch Halogen, Cyano, C₁-C₄-Alkyl, C₁-C₄-Halogenalkyl, C₁-C₄-Alkoxy, C₁-C₄-Halogenalkoxy, Dioxolanyl, Piperidinonyl, Pyrrolidinonyl oder Dihydrodioxazinyl substituiertes Phenyl steht.

Ganz besonders bevorzugt sind neue Verbindungen der Formel (IB-1), worin
- G¹: für N oder C-Halogen (insbesondere CF) steht,
und
- G³: für gegebenenfalls durch Pyrimidinyl substituiertes Pyridyl steht.

Durch Halogen (auch "Halo" abgekürzt) substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom, hervorgehoben für Fluor und Chlor.

Der Rest "Pyrimidyl" wird auch als "Pyrimidinyl" bezeichnet.

Bevorzugt, besonders bevorzugt oder ganz besonders bevorzugt sind Verbindungen, welche jeweils die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind Verbindungen der Formel (I), in welchen eine Kombination der vorstehend als bevorzugt aufgeführten Bedeutungen vorliegt.

Erfindungsgemäß besonders bevorzugt sind Verbindungen der Formel (I), in welchen eine Kombination der vorstehend als besonders bevorzugt aufgeführten Bedeutungen vorliegt.

Erfindungsgemäß ganz besonders bevorzugt sind Verbindungen der Formel (I), in welchen eine Kombination der vorstehend als ganz besonders bevorzugt aufgeführten Bedeutungen vorliegt.

In einer weiteren hervorgehobenen Gruppe von Verbindungen der Formel (IB-1) steht G¹ für N.

In einer weiteren hervorgehobenen Gruppe von Verbindungen der Formel (IB-1) steht G¹ für C-Halogen.

Die Verbindungen der Formel (I) und (IB) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Beispielhaft und ergänzend wird die Herstellung von Verbindungen der Formel (I) in den folgenden Formelschemata erläutert. An dieser Stelle sei auch auf die Herstellungsbeispiele verwiesen. Der Rest G¹ wird in den Schemata auch als G₁ bezeichnet.

In den folgenden Formelschemata kann der Rest R verschiedene Bedeutungen annehmen, die sich, wenn nicht angegeben, aber aus dem jeweiligen Zusammenhang ableiten lassen.

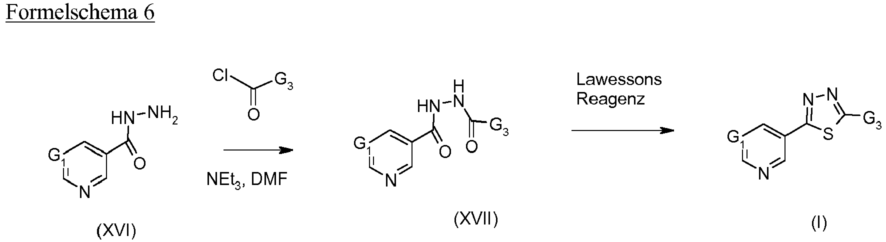

Die erfindungsgemäßen Thiadiazole der Formel (I) gemäß Formelschema 6 werden erhalten, indem Nikotinsäurehydrazide der Formel (XVI) mit beispielsweise Carbonsäurechloriden zu Verbindungen der Formel (XVII) umgesetzt und danach in Gegenwart eines Schwefelungsreagenzes wie Lawessons Reagenz ohne Verdünnungsmittel (vgl. Youji Huaxue (2004), 24(5), 502) oder in einem Verdünnungsmittel wie Toluol oder Anisol cyclisiert werden. Hydrazide der Formel (XVI) sind beschrieben, beispielsweise in Journal of Medicinal Chemistry, 32, 3, 1989, 583-593 und durch Hydrazinolyse der entsprechenden Carbonsäureester erhältlich

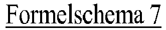

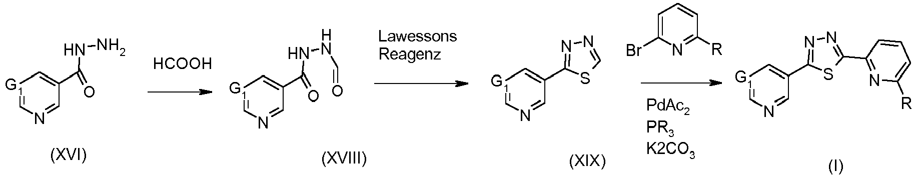

Alternativ zu dem in Formelschema 6 beschriebenen Prozeß können erfindungsgemäße Thiadiazole der Formel (I) auch durch Heck-Reaktion analog dem in Formelschema (I) beschriebenen Verfahren, erhalten werden, siehe dazu Herstellungsbeispiel 10. Die benötigten Thiadiazole der Formel (XIX) können aus Nikotinsäurehydraziden der Formel (XVI) durch Umsetzung mit Ameisensäure in Analogie zu Bull. Soc. Chim. Belg. Vol 106, No 2, 1997, 109 zu den Formylhydraziden der Formel (XVIII) und anschließende Reaktion mit einem Schwefelungsreagenz wie Lawessons Reagenz in einem Verdünnungsmittel wie Toluol oder Anisol erhalten werden, siehe dazu Journal of Indian Chemistry 8, 1970, 509.

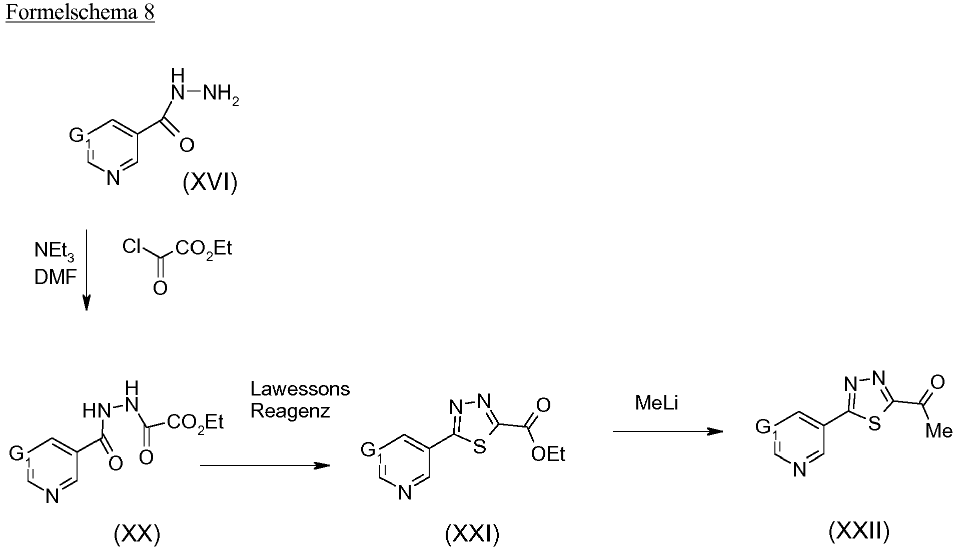

Formelschema 8 beschreibt die Herstellung von Ketonen der Formel (XXII), siehe auch Herstellungsbeispiel 9. Das Hydrazid der Formel (XVI) wird in Gegenwart einer Base wie z.B. Triethylamin in einem Verdünnungsmittel wie beispielsweise Dimethylformamid (DMF) mit Oxalsäurechloridethylester zur Diacylhydrazinverbindung der Formel (XX) umgesetzt, die anschließend mit einem Schwefelungsmittel wie z.B. mit Lawessons Reagenz zum Thiadiazol der Formel (XXI) reagiert, siehe dazu Liquid Crystals Today, Vol 14,1, 2005, 15-18. Aus dem Ester (XXI) kann durch Umsetzung mit einer Metallmethylverbindung wie Methyllithium das Keton (XXII) erhalten werden, siehe dazu Journal of Organic Chemistry 1997, 4774.

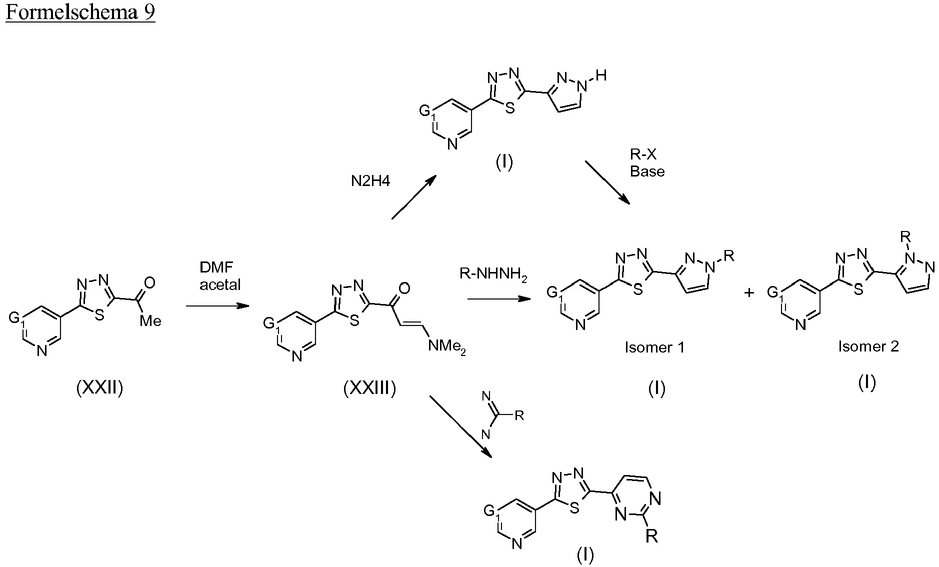

Für Formelschema 9 siehe auch Herstellungsbeispiel 9. Durch Umsetzung des Ketons der Formel (XXII) mit DMF-Acetal werden die Enaminone der Formel (XXIII) analog Formelschema 3 erhalten. Entsprechend dem in Formelschema 4 beschriebenen Verfahren werden aus dem Enaminon der Formel (XXIII) entweder duch Umsetzung mit Hydrazinhydrat die NH-Pyrazole der Formel (I), die sich weiter zu den N-substituierten Pyrazolen der Formel (I) umsetzen lassen oder direkt den N-substituierten Pyrazolen der Formel (I) durch Umsetzung mit substitutiertem Hydrazin erhalten. Die Verbindungen der Formel (I) enstehen in den isomeren Formen Isomer 1 und Isomer 2, wobei der bevorzugte Weg zum Isomer 1 über NH-Pyrazole der Formel (I) führt. Durch Umsetzung des Enaminons der Formel (XXIII) mit Amidinen lassen sich die erfindungsgemäßen Pyrimidine der Formel (I) erhalten.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter

Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen. Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

Die folgenden Beispiele 9 und 10 dienen der Erläuterung der Herstellverfahren, sie sind nicht Gegenstand der Erfindung.

### Beispiel 9:

### Stufe 1: Oxo-[N'-(pyridin-3-carbonyl)-hydrazino]-essigsäureethylester

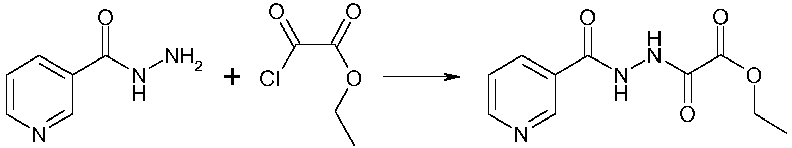

20,5g (149.4mmol) Nikotinsäurehydrazid wurden in 400 ml DMF unter leichtem Erwärmen gelöst und mit 50ml (346mmol) Triethylamin versetzt. Bei Eisbadkühlung versetzte man mit 18ml (160mmol) Oxalsäureethylesterchlorid in 40 ml Dichlormethan mittels eines Tropftrichters. Man rührte noch 15 min bei Raumtemperatur, dann wurde eingedampft. Den Rückstand versetzte man mit aq. Zitronensäure, verd. Natronlauge, aq. Natriumchlorid bis pH=7 und extrahierte 10 mal mit Methylacetat. Die vereinigten organischen Phasen wurden mit Magnesiumsulfat getrocknet und eingedampft.
Ausbeute: 32,5g (91% d. Th), logP (HCOOH) -0,13

### Stufe 2: 5-Pyridin-3-yl-[1,3,4]thiadiazol-2-carbonsäureethylester

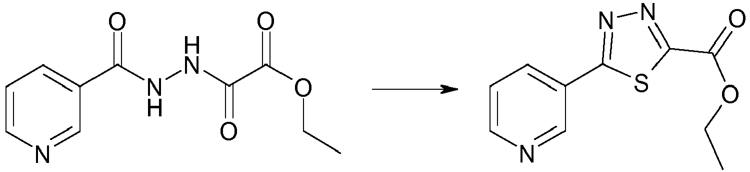

38,1g (160,6mmol) Oxo-[*N*'-(pyridin-3-carbonyl)-hydrazino]-essigsäureethylester wurden mit 37g (91mmol) Lawessons Reagenz in 500 ml Toluol 1h unter Rückfluß erhitzt. Nach Abkühlen wurde eingedampft, mit Ethylacetat, Zitratpuffer bis pH=6 versetzt, vom Ungelösten abfiltriert, die Mischung 4 mal mit Ethylacetat extrahiert; die vereinigten organischen Phasen wurden mit Magnesiumsulfat getrocknet und eingedampft. Den Rückstand kristallisierte man aus Benzotrifluorid um, dabei wurde kurz mit Aktivkohle gekocht und heiß filtriert.
Ausbeute: 16,2g (42% d. Th), logP (HCOOH) 1,41
1H-NMR (D6-DMSO): 1,4 (t, 3H), 4,5 (m, 2H), 7,65 (m, 1H), 8,45 (m, 1H), 8,7 (m, 1H), 9,25 (s, 1H)

### Stufe 3: 1-(5-Pyridin-3-yl-[1,3,4]thiadiazol-2-yl)-ethanon

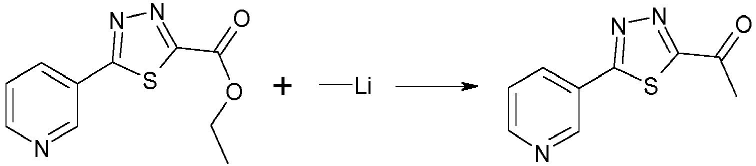

4g (17mmol) 5-Pyridin-3-yl-[1,3,4]thiadiazol-2-carbonsäureethylester wurden in 500ml THF gelöst und bei Trockeineisbadkühlung mit 11,35 ml (34mmol) einer 3-molaren Lösung von Methyllithium versetzt. Man rührte 0,5h, versetzte in der Kälte mit Ethylacetat, dann aq. Zitronensäure; nach Auftauen wurde die Mischung insgesamt dreimal mit Ethylacetat extrahiert. Die vereinigten organischen Phasen wurden mit Magnesiumsulfat getrocknet und eingedampft. Der Rückstand wurde durch Chromatographie an Kieselgel (Cyclohexan / Aceton) gereinigt.
Ausbeute 1,66g (45% d. Th. anteilig berechnet aus 2 Ansätzen), logP (HCOOH) 1,16
1H-NMR (D6-DMSO) 2,75 (s, 3H), 7,6 (m, 1H), 8,45 (d, 1H), 8,8 (d, 1H), 9,2 (s, 1H)

### Stufe 4: (3-Dimethylamino-1-(5-pyridin-3-yl-[1,3,4]thiadiazol-2-yl)-propenon

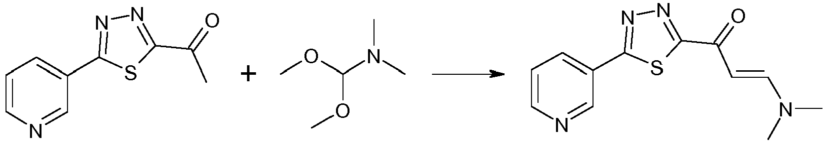

3,48g (17mmol) 1-(5-Pyridin-3-yl-[1,3,4]thiadiazol-2-yl)-ethanon wurden mit 26,6ml (199mmol) DMF-DMA an einer sehr kurzen Destillationsbrücke 2h bei 105°C gerührt, dann 2h bei Siedetemperatur gerührt. Man dampfte ein, der Rückstand wurde an Kieselgel chromatographiert (Cyclohexan/Aceton).
Ausbeute: 1,4g (25% d. Th), logP (HCOOH) 1,05
1H-NMR (CD3CN): 3,0 (s, 3H), 3,2 (s, 3H), 6,1 (d, 1H), 7,5 (m, 1H), 8,0 (m, 1H), 8,3 (m, 1H), 8,7 (m, 1H), 9,2 (s, 1H)

### Stufe 5: 4-(5-Pyridin-3-yl-[1,3,4]thiadiazol-2-yl)-[2,2']bipyrimidinyl

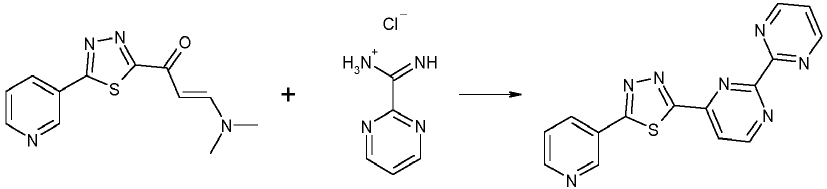

0,3g (1,1mmol) (3-Dimethylamino-1-(5-pyridin-3-yl-[1,3,4]thiadiazol-2-yl)-propenon wurden in 8 ml Ethanol mit 0,18g (1,2 mmol) 2-Amidiniumpyrimidin-Hydrochlorid und 0,4 ml (1,2 mmol) einer 21%igen ethanolischen Natriumethanolat-Lösung 16h unter Rückfluß erhitzt. Die Mischung wurde eingedampft, mit aq. Zitronensäure, aq. Natriumchlorid, verd. Natronlauge bis pH=12 versetzt, mit Chloroform/Isopropanol 10% mehrmals extrahiert, die vereinigten organischen Phasen mit Natriumsulfat getrocknet und eingedampft. Der Rückstand wurde in DMSO/Acetonitril suspendiert, abgesaugt, der Rückstand mit MTBE gewaschen und getrocknet.
Ausbeute: 0,2g (54% d. Th), logP (HCOOH) 1,07
1H-NMR (D6-DMSO) 7,65 (m, 1H), 7,7 (m, 1H), 8,45 (m, 1H), 8,55 (m 1H), 8,8 (m, 1H), 9,1 (m, 2H), 9,3 (m, 2H)

### Beispiel 10:

### Stufe 1: N-Formyl- Nikotinsäurehydrazid

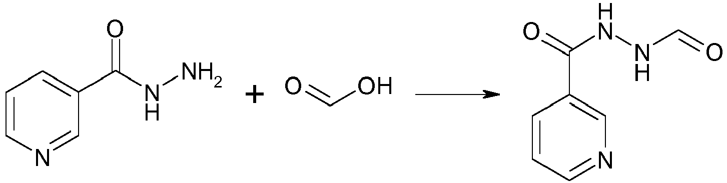

Ausbeute: 28.1g (93% d. Th).
1H-NMR (D6-DMSO): 7,5 (m, 1H), 8-8,3 (m, 2H), 8,75 (m, 1H), 9 (s, 1H), 10 (s, 1H), 10,5 (s, 1H)

### Stufe 2: 3-[1,3,4]Thiadiazol-2-yl-pyridin

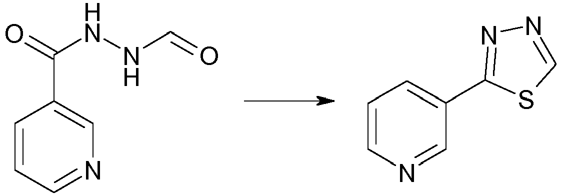

26g (151,3mmol) Formyl-nikotinsäurehydrazid und 45,9g (113,5mmol) Lawessons Reagenz wurden in 500 ml Anisol mit einigen Tropfen Pyridin 16h bei 120°C gerührt. Die Mischung wurde eingedampft, mit aq. Zitronensäure, verd. Natronlauge bis pH=10 versetzt, 1h gerührt und dreimal mit Ethylacetat extrahiert. Die vereinigten organischen Phasen wurden mit Natriumsulfat getrocknet und eingedampft, den Rückstand kristallisierte man aus Benzotrifluorid / Dioxan 5:1 um, dabei wurde mit Aktivkohle gekocht und heiß filtriert.
Ausbeute: 7,6g (30% d. Th), logP (HCOOH) 0,15
1H-NMR (CD3CN) 7,5 (ddd, 1H), 8,35 (m, 1H), 8,7 (d, 1H), 9,15 (m,1H), 9,3 (s, 1H)

### Stufe 3: 2-[6-(5-Pyridin-3-yl-[1,3,4]thiadiazol-2-yl)-pyridin-2-yl]-pyrimidin

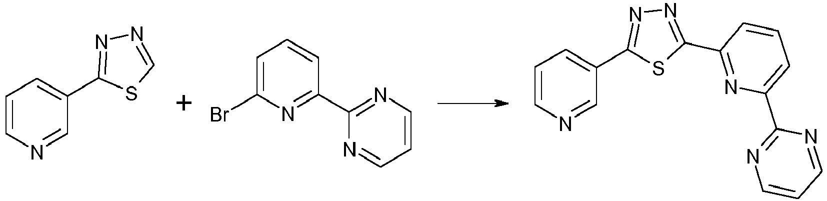

In 60 ml DMF wurden 0,25g (1,1mmol) Palladium(II)acetat unter Argon mit 10,8 ml (4,59mmol) einer 10%igen Lösung von Tri-t-butylphosphan in Hexan versetzt. Mittels Vakuum wurde das Hexan entfernt, dann versetzte man mit 1,5g (9,1mmol) 3-[1,3,4]Thiadiazol-2-yl-pyridin, 2,38g (10,1mmol) 2-(6-Bromo-pyridin-2-yl)-pyrimidin, 2,54g (18,3 mmol) Kaliumcarbonat und ca 0.5 g Tetrabutylammoniumchlorid und rührt 16h bei 130°C. Die Mischung wurde eingedampft, mit aq. Zitronensäure, verdünnter Natronlauge, aq. Natriumchlorid, Ethylacetat bei pH=8 versetzt, mit Ethylacetat dreimal extrahiert; die vereinigten organischen Phasen wurden mit Natriumsulfat getrocknet und eingedampft. Den Rückstand reinigte man durch Chromatographie an Kieselgel
Ausbeute: 0,35g (11%d. Th), logP (HCOOH) 1,54,
1H-NMR (D6-DMSO): 7,6 (m, 2H), 8,25 (t, 1H), 8,45 (m, 2H), 8,55 (m, 1H), 8,75 (m, 1H), 9,05(d, 2H), 9,3 (s, 1H)

In der folgenden Tabelle sind weitere neue erfindungsgemäße Verbindungen aufgeführt.

| **Bsp.Nr.** | **logP ¹⁾ (HCOOH)** | **Formel** | **(M+)+1 (LC/MS)** |
|---|---|---|---|
| 164 | 1,39 | 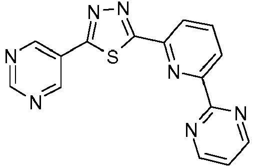 | |
| 165 | 1,91 | 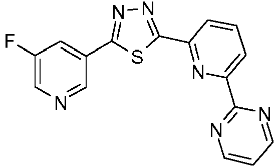 | 337,1 |

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

### Eluenten für die Bestimmung im sauren Bereich (pH 3,4):

Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

**Tabelle 2**

| **Bsp.-Nr.** | **NMR Daten (ppm)** |
|---|---|
| 165 | d₆-DMSO: 7.60 (m,1H), 8.27 (m,1H), 8.42 (m,1H), 8.47 (m,1H), 8.56 (m,1H), 8.78 (m,1H), 9.05 (m,2H), 9.17 (m,1H) |

### Biologische Beispiele

### Beispiel Nr. 1

### Myzus-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. 164, 165

## Patentansprüche

1. Nicht-therapeutische Verwendung von Verbindungen der Formel (IB-1) 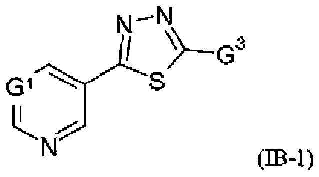 worin
G¹ für N oder C-Halogen steht,
G³ für gegebenenfalls durch Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl, Aminocarbonyl, Pyridyl, oder Pyrimidyl substituiertes Oxazolinyl, Dihydrooxadiazinyl, Dihydrodioxazinyl oder Hydroxypyridyl,
für gegebenenfalls durch Halogen, Nitro, Amino, Alkylamino, Dialkylamino, Alkyl, Halogenalkyl, Cycloalkylalkyl, Alkoxy, Halogenalkoxy, Alkylthio, Alkoxyalkyl, Bis(alkoxy)alkyl, Alkoxycarbonyl, alpha-Hydroxyimino-alkoxycarbonylmethyl, alpha-Alkoxyimino-alkoxycarbonylmethyl, C(X)NR²R³, (worin X für Sauerstoff oder Schwefel steht, R² für Wasserstoff oder Alkyl steht und R³ für Alkyl, Halogenalkyl, Alkoxy, Cyanoalkyl, Alkinyl, Cycloalkyl, Cycloalkylalkyl, Alkoxyalkyl, Alkylthioalkyl oder A-arylalkyl steht oder R² und R³ gemeinsam mit dem Stickstoffatom, an das sie gebunden sind, einen Ring bilden), Alkylthio, Alkylsulfinyl, Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch Alkyl oder Halogenalkyl), Phenyl (welches selbst wiederum substituiert sein kann durch Halogen, Cyano, Nitro, Alkyl oder Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl), die Heteroarylalkylreste Triazolylalkyl, Pyridylalkyl, Pyrimidylalkyl oder Oxadiazolylalkyl (welche selbst wiederum substituiert sein können durch Alkyl) substituiertes Hetaryl aus der Reihe Pyrrolyl, Pyrazolyl, imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, oder
für gegebenenfalls durch Halogen, Cyano, Alkyl, Halogenalkyl, Alkoxy, Halogenalkoxy, Piperidinonyl, Pyrrolidinonyl, Dioxolanyl oder Dihydrodioxazinyl substituiertes Phenyl steht,
sowie von Salzen und N-Oxiden der Verbindungen der Formel (I) zur Bekämpfung von tierischen Schädlingen.

2. Verwendung von Verbindungen der Formel (IB-1) gemäß Anspruch 1, worin
G¹ für N oder C-Halogen steht,
G³ jeweils für gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₁-C₆-Alkylcarbonyl, C₁-C₆-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
für jeweils gegebenenfalls durch Halogen, Nitro, Amino, C₁-C₆-Alkylamino, Di(C₁-C₆-alkyl)amino, C₁-C₆-Alkyl, C₁-C₆-Halogenalkyl, C₃-C₆-Cycloalkyl-C₁-C₆-alkyl, C₁-C₆-Alkoxy, C₁-C₆-Halogenalkoxy, C₁-C₆-Alkylthio, C₁-C₆-Alkoxy-C₁-C₆-alkyl, Bis(C₁-C₆-alkoxy)-C₁-C₆-alkyl, C₁-C₆-Alkoxycarbonyl, alpha-Hydroxyimino-C₁-C₆-alkoxycarbonylmethyl, alpha- C₁-C₆-Alkoxyimino-C₁-C₆-alkoxycarbonylmethyl, C(X)NR²R³, (worin X für Sauerstoff oder Schwefel steht, R² für Wasserstoff oder C₁-C₆-Alkyl steht und R³ für C₁-C₆-Alkyl, C₁-C₆-Halogenalkyl, C₁-C₆-Alkoxy, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkylthio-C₁-C₆-alkyl oder Phenyl-C₁-C₆-alkyl steht), C₁-C₆-Alkylsulfinyl, C₁-C₆-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₆-Alkyl oder C₁-C₆-Halogenalkyl), Phenyl (welches selbst wiederum substituiert sein kann durch Halogen, Cyano, Nitro, C₁-C₆-Alkyl oder C₁-C₆-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl), die Heteroarylalkylreste Triazolyl-C₁-C₆-alkyl, Pyridyl-C₁-C₆-alkyl, Pyrimidyl-C₁-C₆-alkyl oder Oxadiazolyl-C₁-C₆-alkyl (welche selbst wiederum substituiert sein können durch C₁-C₆-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl oder
für gegebenenfalls durch Halogen, Cyano, C₁-C₆-Alkyl, C₁-C₆-Halogenalkyl, C₁-C₆-Alkoxy, C₁-C₆-Halogenalkoxy, Dioxolanyl, Piperidinonyl, Pyrrolidinonyl oder Dihydrodioxazinyl substituiertes Phenyl steht
sowie von Salzen und N-Oxiden der Verbindungen der Formel (IB-1).

3. Verwendung von Verbindungen der Formel (IB-1) gemäß Anspruch 1, worin
G¹ für N oder C-Halogen steht,
G³ jeweils für gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C4-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₁-C₄-Alkylcarbonyl, C₁-C₄-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
für gegebenenfalls durch Halogen, Nitro, Amino, C₁-C₄-Alkylamino, Di-(C₁-C₄-alkyl)amino, C₁-C₄-Alkyl, C₁-C₄-Halogenalkyl, C₃-C₆-Cycloalkyl-C₁-C₄-alkyl, C₁-C₄-Alkoxy, C₁-C₄-Halogenalkoxy, C₁-C₄-Alkylthio, C₁-C₄-Alkoxy-C₁-C₄-alkyl, Bis(C₁-C₄-alkoxy)-C₁-C₄-alkyl, C₁-C₄-Alkoxycarbonyl, alpha-Hydroxyimino-C₁-C₄-alkoxycarbonylmethyl, alpha-C₁-C₄-Alkoxyimino-C₁-C₄-alkoxycarbonylmethyl, C(X)NR²R³, (worin X für Sauerstoff oder Schwefel steht, R² für Wasserstoff oder C₁-C₄-Alkyl steht und R³ für C₁-C₄-Alkyl, C₁-C₄-Halogenalkyl, C₁-C₆-Alkoxy, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht), C₁-C₄-Alkylsulfinyl, C₁-C₄-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₄-Alkyl oder C₁-C₄-Halogenalkyl), Phenyl (welches selbst wiederum substituiert sein kann durch Halogen, Cyano, Nitro, C₁-C₄-Alkyl oder C₁-C₄-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl), die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch C₁-C₄-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl oder
für gegebenenfalls durch Halogen, Cyano, C₁-C₄-Alkyl, C₁-C₄-Halogenalkyl, C₁-C₄-Alkoxy, C₁-C₄-Halogenalkoxy, Dioxolanyl, Piperidinonyl, Pyrrolidinonyl oder Dihydrodioxazinyl substituiertes Phenyl steht
sowie von Salzen und N-Oxiden der Verbindungen der Formel (IB-1).

4. Verwendung von Verbindungen der Formel (IB-1) gemäß Anspruch 1, worin
G¹ für N oder C-Halogen steht,
G³ für gegebenenfalls durch Pyrrolyl oder Pyrimidinyl substituiertes Pyridyl, steht,
sowie von Salzen und N-Oxiden der Verbindungen der Formel (IB-1).

5. Verbindungen der Formel (IB-1) 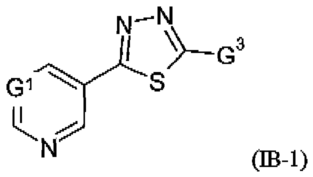 worin
G¹ für N oder C-Halogen steht,
G³ jeweils für gegebenenfalls durch Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl, Aminocarbonyl, Pyridyl oder Pyrimidyl, substituiertes Oxazolinyl, Dihydrooxadiazinyl, Dihydrodioxazinyl oder Hydroxypyridyl,
für gegebenenfalls durch Halogen, Nitro, Amino, Alkylamino, Dialkylamino, Alkyl, Halogenalkyl, Cycloalkylalkyl, Alkoxy, Halogenalkoxy, Alkylthio, Alkoxyalkyl, Bis(alkoxy)alkyl, Alkoxycarbonyl, alpha-Hydroxyimino-alkoxycarbonylmethyl, alpha-Alkoxyimino-alkoxycarbonylmethyl, C(X)NR²R³, (worin X für Sauerstoff oder Schwefel steht, R² für Wasserstoff oder Alkyl steht und R³ für Alkyl, Halogenalkyl, Alkoxy, Cyanoalkyl, Alkinyl, Cycloalkyl, Cycloalkylalkyl, Alkoxyalkyl, Alkylthioalkyl oder Arylalkyl steht), Alkylthio, Alkylsulfinyl, Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch Alkyl oder Halogenalkyl), Phenyl (welches selbst wiederum substituiert sein kann durch Halogen, Cyano, Nitro, Alkyl oder Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl), die Heteroarylalkylreste Triazolylalkyl, Pyridylalkyl, Pyrimidylalkyl oder Oxadiazolylalkyl (welche selbst wiederum substituiert sein können durch Alkyl) substituiertes Hetaryl aus der Reihe Pyrrolyl, Pyrazolyl, Imidazolyl, 1,2,3-Triazolyl, 1,2,4-Triazolyl, Oxazolyl, Isoxazolyl, Triazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, oder
für gegebenenfalls durch Halogen, Cyano, Alkyl, Halogenalkyl, Alkoxy, Halogenalkoxy Dioxolanyl, Piperidinonyl, Pyrrolidinonyl oder Dihydrodioxazinyl substituiertes Phenyl steht.

6. Verbindungen der Formel (IB-1) gemäß Anspruch 5, worin
G¹ für N oder C-Halogen steht,
G³ jeweils für gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsufinyl, C₁-C₆-Alkylsulfonyl, C₁-C₆-Haloalkylsulfinyl, C₁-C₆-Haloalkylsulfonyl, Amino, C₁-C₆-Alkylanmio, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₁-C₆-Alkylcarbonyl, C₁-C₆-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
für jeweils gegebenenfalls durch Halogen, Nitro, Amino, C₁-C₆-Alkylamino, Di-(C₁-C₆-alkyl)amino, C₁-C₆-Alkyl, C₁-C₆-Halogenalkyl, C₃-C₆-Cycloalkyl-C₁-C₆-alkyl, C₁-C₆-Alkoxy, C₁-C₆-Halogenalkoxy, C₁-C₆-Alkylthio, C₁-C₆-Alkoxy-C₁-C₆-alkyl, Bis(C₁-C₆-alkoxy)-C₁-C₆-alkyl, C₁-C₆-Alkoxycarbonyl, alpha-Hydroxyimino-C₁-C₆-alkoxycarbonylmethyl, alpha- C₁-C₆-Alkoxyimino-C₁-C₆-alkoxycarbonylmethyl, C(X)NR²R³, (worin X für Sauerstoff oder Schwefel steht, R² für Wasserstoff oder C₁-C₆-Alkyl steht und R³ für C₁-C₆-Alkyl, C₁-C₆-Halogenalkyl, C₁-C₆-Alkoxy, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkylthio-C₁-C₆-alkyl oder Phenyl-C₁-C₆-alkyl steht), C₁-C₆-Alkylsulfinyl, C₁-C₆-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₆-Alkyl oder C₁-C₆-Halogenalkyl), Phenyl (welches selbst wiederum substituiert sein kann durch Halogen, Cyano, Nitro, C₁-C₆-Alkyl oder C₁-C₆-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl), die Heteroarylalkylreste Triazolyl-C₁-C₆-alkyl, Pyridyl-C₁-C₆-alkyl, Pyrimidyl-C₁-C₆-alkyl oder Oxadiazolyl-C₁-C₆-alkyl (welche selbst wiederum substituiert sein können durch C₁-C₆-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl oder
für gegebenenfalls durch Halogen, Cyano, C₁-C₆-Alkyl, C₁-C₆-Halogenalkyl, C₁-C₆-Alkoxy, C₁-C₆-Halogenalkoxy, Dioxolanyl, Piperidinonyl, Pyrrolidinonyl oder Dihydrodioxazinyl substituiertes Phenyl steht.

7. Verbindungen der Formel (IB-1) gemäß Anspruch 5, worin
G¹ für N oder C-Halogen steht,
G³ jeweils für gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₁-C₄-Alkylcarbonyl, C₁-C₄-Alkoxycarbonyl, Aminocarbonyl, Pyridyl oder Pyrimidyl substituiertes Oxazolinyl, Dihydrooxadiazinyl, Dihydrodioxazinyl oder Hydroxypyridyl,
für jeweils gegebenenfalls durch Halogen, Nitro, Amino, C₁-C₄-Alkylamino, Di-(C₁-C₄-alkyl)amino, C₁-C₄-Alkyl, C₁-C₄-Halogenalkyl, C₃-C₆-Cycloalkyl-C₁-C₄-alkyl, C₁-C₄-Alkoxy, C₁-C₄-Halogenalkoxy, C₁-C₄-Alkylthio, C₁-C₄-Alkoxy-C₁-C₄-alkyl, Bis(C₁-C₄-alkoxy)-C₁-C₄-alkyl, C₁-C₄-Alkoxycarbonyl, alpha-Hydroxyimino-C₁-C₄-alkoxycarbonylmethyl, alpha- C₁-C₄-Alkoxyimino-C₁-C₄-alkoxycarbonylmethyl, C(X)NR²R³, (worin X für Sauerstoff oder Schwefel steht, R² für Wasserstoff oder C₁-C₄-Alkyl steht und R³ für C₁-C₄-Alkyl, C₁-C₄-Halogenalkyl, C₁-C₄-Alkoxy, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C4-alkyl oder Phenyl-C₁-C₄-alkyl steht), C₁-C₄-Alkylsulfinyl, C₁-C₄-Alkylsulfonyl, die Heterocyclylreste Morpholinyl, Triazolinon-yl, Dihydrodioxazinyl, Dihydrooxadiazinyl, Dioxolanyl, Dioxanyl, Piperidinonyl, Pyrrolidinonyl und Pyrazolinon-yl (welche selbst wiederum substituiert sein können durch C₁-C₄-Alkyl oder C₁-C₄-Halogenalkyl), Phenyl (welches selbst wiederum substituiert sein kann durch Halogen, Cyano, Nitro, C₁-C₄-Alkyl oder C₁-C₄-Halogenalkyl), die Heteroarylreste Pyrrolyl, Pyridyl, Pyridyl-N-oxid, Pyrimidyl, Imidazolyl, Pyrazolyl, Oxazolyl, Thiazolyl, Furanyl, Thienyl, Thiazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl), die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch C₁-C₄-Alkyl) substituiertes Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl oder
für gegebenenfalls durch Halogen, Cyano, C₁-C₄-Alkyl, C₁-C₄-Halogenalkyl, C₁-C₄-Alkoxy, C₁-C₄-Halogenalkoxy, Dioxolanyl, Piperidinonyl, Pyrrolidinonyl oder Dihydrodioxazinyl substituiertes Phenyl steht.

8. Verbindungen der Formel (IB-1) gemäß Anspruch 5, worin G¹ für N oder C-Halogen steht und G³ für gegebenenfalls durch Pyrimidinyl substituiertes Pyridyl steht.

9. Mittel, **gekennzeichnet durch** einen Gehalt von mindestens einer Verbindung der Formel (IB-1) gemäß Ansprüchen 5 bis 8.

10. Nicht-therapeutisches Verfahren zum Bekämpfen von Schädlingen **dadurch gekennzeichnet, dass** man eine Verbindung der Formel (IB-1) gemäß Ansprüchen 5 bis 8 oder ein Mittel gemäß Anspruch 9 auf die Schädlinge und/oder ihren Lebensraum einwirken lässt.

## Claims

1. The non-therapeutic use of compounds of the formula (IB-I) 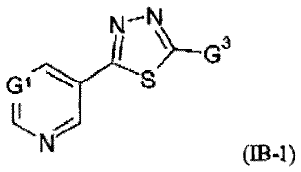 in which
G¹ represents N or C-halogen,
G³ represents oxazolinyl, dihydrooxadiazinyl, dihydrodioxazinyl or hydroxypyridyl which is optionally substituted by halogen, cyano, nitro, alkyl, halogenalkyl, cycloalkyl, alkoxy, haloalkoxy, alkylthio, haloalkylthio, alkylsulfinyl, alkylsulfonyl, haloalkylsulfinyl, haloalkylsulfonyl, amino, alkylamino, dialkylamino, alkylcarboxylamino, alkoxy-carbonylamino, alkoxyalkyl, haloalkoxyalkyl, alkenyl, alkynyl, alkylcycloalkyl, alkylcarbonyl, alkoxycarbonyl, aminocarbonyl, pyridyl or pyrimidyl,
represents hetaryl from the group consisting of pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4,oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiozolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl optionally substituted by halogen, nitro, amino, alkylamino, dialkylamino, alkyl, haloalkyl, cycloalkylalkyl, alkoxy, haloalkoxy, alkylthio, alkoxyalkyl, bis(alkoxy)alkyl, alkoxycarbonyl, alpha-hydroxyiminoalkoxycarbonylmethyl, alphalkoxyiminoalkoxycarbonylmethyl, C(X)NR²R³ (in which X represents oxygen or sulphur, R² represents hydrogen or alkyl and R³ represents alkyl, haloalkyl, alkoxy, cyanoalkyl, alkynyl, cycloalkyl, cycloalkylalkyl, alkoxyalkyl, alkylthioalkyl or arylalkyl or R² and R³ together with the nitrogen atom to which they are attached form a ring), alkylthio, alkylsulfinyl, alkylsulfonyl, the heterocyclyl radicals morpholinyl, triazolinonyl, dihydrodioxazinyl, dihydrooxadiazinyl, dioxolanyl, dioxanyl, piperidinonyl, pyrrolidinonyl and pyrazolinonyl (which for their part may be substituted by alkyl or haloalkyl), phenyl (which for its part may be substituted by halogen, cyano, nitro, alkyl or haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, alkyl, haloalkyl, alkoxy, haloalkoxy, alkoxyalkyl, alkylthio, alkylthioalkyl and cycloalkyl), the heteroarylalkyl radicals triazolylalkyl, pyridylalkyl, pyrimidylalkyl or oxadiazolyalkyl (which for their part may be substituted by alkyl), or
represents phenyl which is optionally substituted by halogen, cyano, alkyl, haloalkyl, alkoxy, haloalkoxy, piperidinonyl, pyrrolidinonyl, dioxolanyl or dihydrodioxazinyl,
and of salts and N-oxides of the compounds of the formula (I) for controlling animal pests.

2. Use of compounds of the formula (IB-I) according to Claim 1 in which
G¹ represents N or C-halogen,
G³ represents oxazolinyl, dihydrooxadiazinyl, dihydrodioxazinyl or hydroxypyridyl, each of which is optionally substituted by halogen, cyano, nitro, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-alkylsulfonyl, C₁-C₆-haloalkylsulfinyl, C₁-C₆-haloalkylsulfonyl, amino, C₁-C₆-alkylamino, di-(C₁-C₆-alkyl)amino, C₁-C₆-alkylcarbonylamino, C₁-C₆-alkoxycarbonylamino, C₁-C₆-alkoxy-C₁-C₆-alkyl, C₁-C₆-haloalkoxy-C₁-C₆-alkyl, C₂-C₆-alkenyl, C₂-C₆-alkynyl, C₁-C₆-alkyl-C₃-C₆-cycloalkyl, C₁-C₆-alkylcarbonyl, C₁-C₆-alkoxycarbonyl, aminocarbonyl, pyridyl or pyrimidyl,
represents pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl, each of which is optionally substituted by halogen, nitro, amino, C₁-C₆-alkylamino, di-(C₁-C₆-alkyl)amino, C₁-C₆-alkyl, C₁-C₆-haloalkyl, C₃-C₆-cycloalkyl-C₁-C₆-alkyl, C₁-C₆-alkoxy, C₁-C₆-haloalkoxy, C₁-C₆-alkylthio, C₁-C₆-alkoxy-C₁-C₆-alkyl, bis(C₁-C₆-alkoxy)-C₁-C₆-alkyl, C₁-C₆-alkoxycarbonyl, alpha-hydroxyimino-C₁-C₆-alkoxycarbonylmethyl, alpha-C₁-C₆-alkoxyimino-C₁-C₆-alkoxycarbonylmethyl, C(X)NR²R³, (in which X represents oxygen or sulphur, R² represents hydrogen or C₁-C₆-alkyl and R³ represents C₁-C₆-alkyl, C₁-C₆-haloalkyl, C₁-C₆-alkoxy, cyano-C₁-C₆-alkyl, C₂-C₆-alkynyl, C₃-C₆-cycloalkyl, C₃-C₆-cycloalkyl-C₁-C₆-alkyl, C₁-C₆-alkoxy-C₁-C₆-alkyl, C₁-C₆-alkylthio-C₁-C₆-alkyl or phenyl-C₁-C₆-alkyl), C₁-C₆-alkylsulfinyl, C₁-C₆-alkylsulfonyl, the heterocyclyl radicals morpholinyl, triazolinonyl, dihydrodioxazinyl,dihydrooxadiazinyl, dioxolanyl, dioxanyl, piperidinonyl, pyrrolidinonyl and pyrazolinonyl (which for their part may be substituted by C₁-C₆-alkyl or C₁-C₆-haloalkyl), phenyl (which for its part may be substituted by halogen, cyano, nitro, C₁-C₆-alkyl or C₁-C₆-haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, C₁-C₆-alkyl, C₁-C₆-haloalkyl, C₁-C₆-alkoxy, C₁-C₆-haloalkoxy, C₁-C₆-alkoxy-C₁-C₆-alkyl, C₁-C₆-alkylthio, C₁-C₆-alkylthio-C₁-C₆-alkyl and C₃-C₆-cycloalkyl), the heteroarylalkyl radicals triazolyl-C₁-C₆-alkyl, pyridyl-C₁-C₆-alkyl, pyrimidyl-C₁-C₆-alkyl or oxadiazolyl-C₁-C₆-alkyl (which for their part may be substituted by C₁-C₆-alkyl) or
represents phenyl which is optionally substituted by halogen, cyano, C₁-C₆-alkyl, C₁-C₆-haloalkyl, C₁-C₆-alkoxy, C₁-C₆-haloalkoxy, dioxolanyl, piperidinonyl, pyrrolidinonyl or dihydro dioxazinyl
and of salts and N-oxides of the compounds of the formula (IB-I).

3. Use of compounds of the formula (IB-I) according to Claim 1 in which
G¹ represents N or C-halogen,
G³ represents oxazolinyl, dihydrooxadiazinyl, dihydrodioxazinyl or hydroxypyridyl, each of which is optionally substituted by halogen, cyano, nitro, C₁-C₄-alkyl, C₁-C₄-haloalkyl, C₃-C₆-cycloalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-alkylsulfonyl, C₁-C₄-haloalkylsulfinyl, C₁-C₄-haloalkylsulfonyl, amino, C₁-C₄-alkylamino, di-(C₁-C₄-alkyl) amino, C₁-C₄-alkylcarbonylamino, C₁-C₄-alkoxycarbonylamino, C₁-C₄-alkoxy-C₁-C₄-alkyl, C₁-C₄-haloalkoxy-C₁-C₄-alkyl, C₂-C₄-alkenyl, C₂-C₄-alkynyl, C₁-C₄-alkyl-C₃-C₆-cycloalkyl, C₁-C₄-alkylcarbonyl, C₁-C₄-alkoxycarbonyl, aminocarbonyl, pyridyl or pyrimidyl,
represents pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl which is optionally substituted by halogen, nitro, amino, C₁-C₄-alkylamino, di-(C₁-C₄-alkyl) amino, C₁-C₄-alkyl, C₁-C₄-haloalkyl, C₃-C₆-cycloalkyl-C₁-C₄-alkyl, C₁-C₄-alkoxy, C₁-C₄-haloalkoxy, C₁-C₄-alkylthio, C₁-C₄-alkoxy-C₁-C₄-alkyl, bis (C₁-C₄-alkoxy) -C₁-C₄-alkyl, C₁-C₄-alkoxycarbonyl, alpha-hydroxyimino-C₁-C₄-alkoxycarbonylmethyl, alpha-C₁-C₄-alkoxyimino-C₁-C₄-alkoxycarbonylmethyl, C(X)NR²R³, (in which X represents oxygen or sulphur, R² represents hydrogen or C₁-C₄-alkyl and R³ represents C₁-C₄-alkyl, C₁-C₄-haloalkyl, C₁-C₆-alkoxy, cyano-C₁-C₄-alkyl, C₂-C₄-alkinyl, C₃-C₆-cycloalkyl, C₃-C₆-cycloalkyl-C₁-C₄-alkyl, C₁-C₄-alkoxy-C₁-C₄-alkyl, C₁-C₄-alkylthio-C₁-C₄-alkyl or phenyl-C₁-C₄-alkyl) , C₁-C₄-alkylsulfinyl, C₁-C₄-alkylsulfonyl, the heterocyclyl radicals morpholinyl, triazolinonyl, dihydrodioxazinyl,dihydrooxadiazinyl, dioxolanyl, dioxanyl, piperidinonyl, pyrrolidinonyl and pyrazolinonyl (which for their part may be substituted by C₁-C₄-alkyl or C₁-C₄-haloalkyl), phenyl (which for its part may be substituted by halogen, cyano, nitro, C₁-C₄-alkyl or C₁-C₄-haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, C₁-C₄-alkyl, C₁-C₄-haloalkyl, C₁-C₄-alkoxy, C₁-C₄-haloalkoxy, C₁-C₄-alkoxy-C₁-C₄-alkyl, C₁-C₄-alkylthio, C₁-C₄-alkylthio-C₁-C₄-alkyl and C₃-C₆-cycloalkyl), the heteroarylalkyl radicals triazolyl-C₁-C₄-alkyl, pyridyl-C₁-C₄-alkyl, pyrimidyl-C₁-C₄-alkyl or oxadiazolyl-C₁-C₄-alkyl (which for their part may be substituted by C₁-C₄-alkyl) or
represents phenyl which is optionally substituted by halogen, cyano, C₁-C₄-alkyl, C₁-C₄-haloalkyl, C₁-C₄-alkoxy, C₁-C₄-haloalkoxy, dioxolanyl, piperidinonyl, pyrrolidinonyl or dihydrodioxazinyl
and of salts and N-oxides of the compounds of the formula (IB-I).

4. Use of compounds of the formula (IB-I) according to Claim 1 in which
G¹ represents N or C-halogen,
G³ represents pyridyl which is optionally substituted by pyrrolyl or pyrimidinyl,
and of salts and N-oxides of the compounds of the formula (IB-I).

5. Compounds of the formula (IB-I) 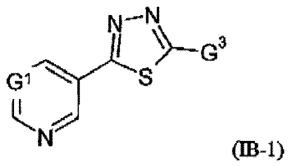 in which
G¹ represents N or C-halogen,
G³ represents oxazolinyl, dihydrooxadiazinyl, dihydrodioxazinyl or hydroxypyridyl, each of which is optionally substituted by halogen, cyano, nitro, alkyl, haloalkyl, cycloalkyl, alkoxy, haloalkoxy, alkylthio, haloalkylthio, alkylsulfinyl, alkylsulfonyl, haloalkylsulfinyl, haloalkylsulfonyl, amino, alkylamino, dialkylamino, alkylcarbonylamino, alkoxy-carbonylamino, alkoxyalkyl, haloalkoxyalkyl, alkenyl, alkynyl, alkylcycloalkyl, alkylcarbonyl, alkoxycarbonyl, aminocarbonyl, pyridyl or pyrimidyl,
represents hetaryl from the group consisting of pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4,oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiozolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl optionally substituted by halogen, nitro, amino, alkylamino, dialkylamino, alkyl, haloalkyl, cycloalkylalkyl, alkoxy, haloalkoxy, alkylthio, alkoxyalkyl, bis(alkoxy)alkyl, alkoxycarbonyl, alpha-hydroxyiminoalkoxycarbonylmethyl, alphalkoxyiminoalkoxycarbonylmethyl, C (X) NR²R³ (in which X represents oxygen or sulphur, R² represents hydrogen or alkyl and R³ represents alkyl, haloalkyl, alkoxy, cyanoalkyl, alkynyl, cycloalkyl, cycloalkylalkyl, alkoxyalkyl, alkylthioalkyl or arylalkyl), alkylthio, alkylsulfinyl, alkylsulfonyl, the heterocyclyl radicals morpholinyl, triazolinonyl, dihydrodioxazinyl, dihydrooxadiazinyl, dioxolanyl, dioxanyl, piperidinonyl, pyrrolidinonyl and pyrazolinonyl (which for their part may be substituted by alkyl or haloalkyl), phenyl (which for its part may be substituted by halogen, cyano, nitro, alkyl or haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, alkyl, haloalkyl, alkoxy, haloalkoxy, alkoxyalkyl, alkylthio, alkylthioalkyl and cycloalkyl), the heteroarylalkyl radicals triazolylalkyl, pyridylalkyl, pyrimidylalkyl or oxadiazolylalkyl (which for their part may be substituted by alkyl), or
represents phenyl which is optionally substituted by halogen, cyano, alkyl, haloalkyl, alkoxy, haloalkoxy, dioxolanyl, piperidinonyl, pyrrolidinonyl or dihydrodioxazinyl.

6. Compounds of the formula (IB-I) according to Claim 5 in which
G¹ represents N or C-halogen,
G³ represents oxazolinyl, dihydrooxadiazinyl, dihydrodioxazinyl or hydroxypyridyl, each of which is optionally substituted by halogen, cyano, nitro, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-alkylsulfonyl, C₁-C₆-haloalkylsulfinyl, C₁-C₆-haloalkylsulfonyl, amino, C₁-C₆-alkylamino, di-(C₁-C₆-alkyl)amino, C₁-C₆-alkylcarbonylamino, C₁-C₆-alkoxycarbonylamino, C₁-C₆-alkoxy-C₁-C₆-alkyl, C₁-C₆-haloalkoxy-C₁-C₆-alkyl, C₂-C₆-alkenyl, C₂-C₆-alkynyl, C₁-C₆-alkyl-C₃-C₆-cycloalkyl, C₁-C₆-alkylcarbonyl, C₁-C₆-alkoxycarbonyl, aminocarbonyl, pyridyl or pyrimidyl,
represents pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl each of which is optionally substituted by halogen, nitro, amino, C₁-C₆-alkylamino, di-(C₁-C₆-alkyl)amino, C₁-C₆-alkyl, C₁-C₆-haloalkyl, C₃-C₆-cycloalkyl-C₁-C₆-alkyl, C₁-C₆-alkoxy, C₁-C₆-haloalkoxy, C₁-C₆-alkylthio, C₁-C₆-alkoxy-C₁-C₆-alkyl, bis (C₁-C₆-alkoxy) -C₁-C₆-alkyl, C₁-C₆-alkoxycarbonyl, alpha-hydroxyimino-C₁-C₆-alkoxycarbonylmethyl, alpha-C₁-C₆-alkoxyimino-C₁-C₆-alkoxycarbonylmethyl, C (X) NR²R³, (in which X represents oxygen or sulphur, R² represents hydrogen or C₁-C₆-alkyl and R³ represents C₁-C₆-alkyl, C₁-C₆-haloalkyl, C₁-C₆-alkoxy, cyano-C₁-C₆-alkyl, C₂-C₆-alkynyl, C₃-C₆-cycloalkyl, C₃-C₆-cycloalkyl-C₁-C₆-alkyl, C₁-C₆-alkoxy-C₁-C₆-alkyl, C₁-C₆-alkylthio-C₁-C₆-alkyl or phenyl-C₁-C₆-alkyl), C₁-C₆-alkylsulfinyl, C₁-C₆-alkylsulfonyl, the heterocyclyl radicals morpholinyl, triazolinonyl, dihydrodioxazinyl,dihydrooxadiazinyl, dioxolanyl, dioxanyl, piperidinonyl, pyrrolidinonyl and pyrazolinonyl (which for their part may be substituted by C₁-C₆-alkyl or C₁-C₆-haloalkyl) , phenyl (which for its part may be substituted by halogen, cyano, nitro, C₁-C₆-alkyl or C₁-C₆-haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, C₁-C₆-alkyl, C₁-C₆-haloalkyl, C₁-C₆-alkoxy, C₁-C₆-haloalkoxy, C₁-C₆-alkoxy-C₁-C₆-alkyl, C₁-C₆-alkylthio, C₁-C₆-alkylthio-C₁-C₆-alkyl and C₃-C₆-cycloalkyl), the heteroarylalkyl radicals triazolyl-C₁-C₆-alkyl, pyridyl-C₁-C₆-alkyl, pyrimidyl-C₁-C₆-alkyl or oxadiazolyl-C₁-C₆-alkyl (which for their part may be substituted by C₁-C₆-alkyl) or
represents phenyl which is optionally substituted by halogen, cyano, C₁-C₆-alkyl, C₁-C₆-haloalkyl, C₁-C₆-alkoxy, C₁-C₆-haloalkoxy, dioxolanyl, piperidinonyl, pyrrolidinonyl or dihydrodioxazinyl.

7. Compounds of the formula (IB-I) according to Claim 5 in which
G¹ represents N or C-halogen,
G³ represents oxazolinyl, dihydrooxadiazinyl, dihydrodioxazinyl or hydroxypyridyl, each of which is optionally substituted by halogen, cyano, nitro, C₁-C₄-alkyl, C₁-C₄-haloalkyl, C₃-C₆-cycloalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-alkylsulfonyl, C₁-C₄-haloalkylsulfinyl, C₁-C₄-haloalkylsulfonyl, amino, C₁-C₄-alkylamino, di-(C₁-C₄-alkyl) amino, C₁-C₄-alkylcarbonylamino, C₁-C₄-alkoxycarbonylamino, C₁-C₄-alkoxy-C₁-C₄-alkyl, C₁-C₄-haloalkoxy-C₁-C₄-alkyl, C₂-C₄-alkenyl, C₂-C₄-alkynyl, C₁-C₄-alkyl-C₃-C₆-cycloalkyl, C₁-C₄-alkylcarbonyl, C₁-C₄-alkoxycarbonyl, aminocarbonyl, pyridyl or pyrimidyl,
represents pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl, each of which is optionally substituted by halogen, nitro, amino, C₁-C₄-alkylamino, di-(C₁-C₄-alkyl) amino, C₁-C₄-alkyl, C₁-C₄-haloalkyl, C₃-C₆-cycloalkyl-C₁-C₄-alkyl, C₁-C₄-alkoxy, C₁-C₄-haloalkoxy, C₁-C₄-alkylthio, C₁-C₄-alkoxy-C₁-C₄-alkyl, bis (C₁-C₄-alkoxy) -C₁-C₄-alkyl, C₁-C₄-alkoxycarbonyl, alpha-hydroxyimino-C₁-C₄-alkoxycarbonylmethyl, alpha-C₁-C₄-alkoxyimino-C₁-C₄-alkoxycarbonylmethyl, C (X) NR²R³, (in which X represents oxygen or sulphur, R² represents hydrogen or C₁-C₄-alkyl and R³ represents C₁-C₄-alkyl, C₁-C₄-haloalkyl, C₁-C₄-alkoxy, cyano-C₁-C₄-alkyl, C₂-C₄-alkinyl, C₃-C₆-cycloalkyl, C₃-C₆-cycloalkyl-C₁-C₄-alkyl, C₁-C₄-alkoxy-C₁-C₄-alkyl, C₁-C₄-alkylthio-C₁-C₄-alkyl or phenyl-C₁-C₄-alkyl), C₁-C₄-alkylsulfinyl, C₁-C₄-alkylsulfonyl, the heterocyclyl radicals morpholinyl, triazolinonyl, dihydrodioxazinyl,dihydrooxadiazinyl, dioxolanyl, dioxanyl, piperidinonyl, pyrrolidinonyl and pyrazolinonyl (which for their part may be substituted by C₁-C₄-alkyl or C₁-C₄-haloalkyl), phenyl (which for its part may be substituted by halogen, cyano, nitro, C₁-C₄-alkyl or C₁-C₄-haloalkyl), the heteroaryl radicals pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for their part may be substituted by halogen, nitro, C₁-C₄-alkyl, C₁-C₄-haloalkyl, C₁-C₄-alkoxy, C₁-C₄-haloalkoxy, C₁-C₄-alkoxy-C₁-C₄-alkyl, C₁-C₄-alkylthio, C₁-C₄-alkylthio-C₁-C₄-alkyl and C₃-C₆-cycloalkyl), the heteroarylalkyl radicals triazolyl-C₁-C₄-alkyl, pyridyl-C₁-C₄-alkyl, pyrimidyl-C₁-C₄-alkyl or oxadiazolyl-C₁-C₄-alkyl (which for their part may be substituted by C₁-C₄-alkyl) or
represents phenyl which is optionally substituted by halogen, cyano, C₁-C₄-alkyl, C₁-C₄-haloalkyl, C₁-C₄-alkoxy, C₁-C₄-haloalkoxy, dioxolanyl, piperidinonyl, pyrrolidinonyl or dihydrodioxazinyl.

8. Compounds of the formula (IB-I) according to Claim 5 in which G¹ represents N or C-halogen
and
G³ represents pyridyl which is optionally substituted by pyrimidinyl.

9. Compositions, **characterized in that** they comprise at least one compound of the formula (IB-I) according to Claims 5 to 8.

10. Non-theraputic method for controlling pests, **characterized in that** a compound of the formula (IB-I) according to Claims 5 to 8 or a composition according to Claim 9 is allowed to act on the pests and/or their habitat.

## Revendications

1. Utilisation non-thérapeutique de composés de formule (IB-1) 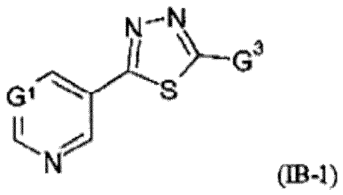 où
G¹ représente N ou C-halogène,
G³ représente oxazolinyle, dihydrooxadiazinyle, dihydrodioxazinyle ou hydroxypyridyle, le cas échéant substitué par halogène, cyano, nitro, alkyle, halogénoalkyle, cycloalkyle, alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylcarbonylamino, alcoxycarbonylamino, alcoxyalkyle, halogénoalcoxyalkyle, alcényle, alcynyle, alkylcycloalkyle, alkylcarbonyle, alcoxycarbonyle, aminocarbonyle, pyridyle ou pyrimidyle,
hétaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle, le cas échéant substitué par halogène, nitro, amino, alkylamino, dialkylamino, alkyle, halogénoalkyle, cycloalkylalkyle, alcoxy, halogénoalcoxy, alkylthio, alcoxyalkyle, bis(alcoxy)alkyle, alcoxycarbonyle, alpha-hydroxyiminoalcoxycarbonylméthyle, alpha-alcoxyiminoalcoxycarbonylméthyle, C (X) NR²R³, (où X représente oxygène ou soufre, R² représente hydrogène ou alkyle et R³ représente alkyle, halogénoalkyle, alcoxy, cyanoalkyle, alcynyle, cycloalkyle, cycloalkylalkyle, alcoxyalkyle, alkylthioalkyle ou arylalkyle ou R² et R³ forment, ensemble avec l'atome d'azote auquel ils sont liés, un cycle), alkylthio, alkylsulfinyle, alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par alkyle ou halogénoalkyle), phényle (qui peut à son tour être substitué par halogène, cyano, nitro, alkyle ou halogénoalkyle), les radicaux hétéroaryle pyrrolyle, pyridyle, pyridyl-N-oxyde, pyrimidyle, imidazolyle, pyrazolyle, oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, alkyle, halogénoalkyle, alcoxy, halogénoalcoxy, alcoxyalkyle, alkylthio, alkylthioalkyle et cycloalkyle), les radicaux hétéroarylalkyle triazolylalkyle, pyridylalkyle, pyrimidylalkyle ou oxadiazolylalkyle (qui peuvent à leur tour être substitués par alkyle) ou
phényle le cas échéant substitué par halogène, cyano, alkyle, halogénoalkyle, alcoxy, halogénoalcoxy, pipéridinonyle, pyrrolidinonyle, dioxolanyle ou dihydrodioxazinyle,
ainsi que de sels et de N-oxydes des composés de formule (I) pour lutter contre des organismes nuisibles animaux.

2. Utilisation de composés de formule (IB-1) selon la revendication 1, dans laquelle
G¹ représente N ou C-halogène,
G³ représente à chaque fois oxazolinyle, dihydrooxadiazinyle, dihydrodioxazinyle ou hydroxypyridyle, le cas échéant substitué par halogène, cyano, nitro, C₁-C₆-alkyle, C₁-C₆-halogénoalkyle, C₃-C₆-cycloalkyle, C₁-C₆-alcoxy, C₁-C₆-halogénoalcoxy, C₁-C₆-alkylthio, C₁-C₆-halogénoalkylthio, C₁-C₆-alkylsulfinyle, C₁-C₆-alkylsulfonyle, C₁-C₆-halogénoalkylsulfinyle, C₁-C₆-halogénoalkylsulfonyle, amino, C₁-C₆-alkylamino, di-(C₁-C₆-alkyl)amino, C₁-C₆-alkylcarbonylamino, C₁-C₆-alcoxycarbonylamino, C₁-C₆-alcoxy-C₁-C₆-alkyle, C₁-C₆-halogénoalcoxy-C₁-C₆-alkyle, C₂-C₆-alcényle, C₂-C₆-alcynyle, C₁-C₆-alkyl-C₃-C₆-cycloalkyle, C₁-C₆-alkylcarbonyle, C₁-C₆-alcoxycarbonyle, aminocarbonyle, pyridyle ou pyrimidyle,
pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle, le cas échéant substitué par halogène, nitro, amino, C₁-C₆-alkylamino, di-(C₁-C₆-alkyl)amino, C₁-C₆-alkyle, C₁-C₆-halogénoalkyle, C₃-C₆-cycloalkyl-C₁-C₆-alkyle, C₁-C₆-alcoxy, C₁-C₆-halogénoalcoxy, C₁-C₆-alkylthio, C₁-C₆-alcoxy-C₁-C₆-alkyle, bis(C₁-C₆-alcoxy)-C₁-C₆-alkyle, C₁-C₆-alcoxycarbonyle, alpha-hydroxyimino-C₁-C₆-alcoxycarbonylméthyle, alpha-C₁-C₆-alcoxyimino-C₁-C₆-alcoxycarbonylméthyle, C (X) NR²R³, (où X représente oxygène ou soufre, R² représente hydrogène ou C₁-C₆-alkyle et R³ représente C₁-C₆-alkyle, C₁-C₆-halogénoalkyle, C₁-C₆-alcoxy, cyano-C₁-C₆-alkyle, C₂-C₆-alcynyle, C₃-C₆-cycloalkyle, C₃-C₆-cycloalkyl-C₁-C₆-alkyle, C₁-C₆-alcoxy-C₁-C₆-alkyle, C₁-C₆-alkylthio-C₁-C₆-alkyle ou phényl-C₁-C₆-alkyle), C₁-C₆-alkylsulfinyle, C₁-C₆-alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par C₁-C₆-alkyle ou C₁-C₆-halogénoalkyle), phényle (qui peut à son tour être substitué par halogène, cyano, nitro, C₁-C₆-alkyle ou C₁-C₆-halogénoalkyle), les radicaux hétéroaryle pyrrolyle, pyridyle, pyridyl-N-oxyde, pyrimidyle, imidazolyle, pyrazolyle, oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, C₁-C₆-alkyle, C₁-C₆-halogénoalkyle, C₁-C₆-alcoxy, C₁-C₆-halogénoalcoxy, C₁-C₆-alcoxy-C₁-C₆-alkyle, C₁-C₆-alkylthio, C₁-C₆-alkylthio-C₁-C₆-alkyle et C₃-C₆-cycloalkyle), les radicaux hétéroarylalkyle triazolyl-C₁-C₆-alkyle, pyridyl-C₁-C₆-alkyle, pyrimidyl-C₁-C₆-alkyle ou oxadiazolyl-C₁-C₆-alkyle (qui peuvent à leur tour être substitués par C₁-C₆-alkyle) ou
phényle le cas échéant substitué par halogène, cyano, C₁-C₆-alkyle, C₁-C₆-halogénoalkyle, C₁-C₆-alcoxy, C₁-C₆-halogénoalcoxy, dioxolanyle, pipéridinonyle, pyrrolidinonyle ou dihydrodioxazinyle
ainsi que de sels et de N-oxydes des composés de formule (IB-1).

3. Utilisation de composés de formule (IB-1) selon la revendication 1, dans laquelle
G¹ représente N ou C-halogène,
G³ représente à chaque fois oxazolinyle, dihydrooxadiazinyle, dihydrodioxazinyle ou hydroxypyridyle, le cas échéant substitué par halogène, cyano, nitro, C₁-C₄-alkyle, C₁-C₄-halogénoalkyle, C₃-C₆-cycloalkyle, C₁-C₄-alcoxy, C₁-C₄-halogénoalcoxy, C₁-C₄-alkylthio, C₁-C₄-halogénoalkylthio, C₁-C₄-alkylsulfinyle, C₁-C₄-alkylsulfonyle, C₁-C₄-halogénoalkylsulfinyle, C₁-C₄-halogénoalkylsulfonyle, amino, C₁-C₄-alkylamino, di-(C₁-C₄-alkyl)amino, C₁-C₄-alkylcarbonylamino, C₁-C₄-alcoxycarbonylamino, C₁-C₄-alcoxy-C₁-C₄-alkyle, C₁-C₄-halogénoalcoxy-C₁-C₄-alkyle, C₂-C₄-alcényle, C₂-C₄-alcynyle, C₁-C₄-alkyl-C₃-C₆-cycloalkyle, C₁-C₄-alkylcarbonyle, C₁-C₄-alcoxycarbonyle, aminocarbonyle, pyridyle ou pyrimidyle
pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle le cas échéant substitué par halogène, nitro, amino, C₁-C₄-alkylamino, di-(C₁-C₄-alkyl)amino, C₁-C₄-alkyle, C₁-C₄-halogénoalkyle, C₃-C₆-cycloalkyl-C₁-C₄-alkyle, C₁-C₄-alcoxy, C₁-C₄-halogénoalcoxy, C₁-C₄-alkylthio, C₁-C₄-alcoxy-C₁-C₄-alkyle, bis (C₁-C₄-alcoxy)-C₁-C₄-alkyle, C₁-C₄-alcoxycarbonyle, alpha-hydroxyimino-C₁-C₄-alcoxycarbonylméthyle, alpha-C₁-C₄-alcoxyimino-C₁-C₄-alcoxycarbonylméthyle, C(X)NR²R³, (où X représente oxygène ou soufre, R² représente hydrogène ou C₁-C₄-alkyle et R³ représente C₁-C₄-alkyle, C₁-C₄-halogénoalkyle, C₁-C₆-alcoxy, cyano-C₁-C₄-alkyle, C₂-C₄-alcynyle, C₃-C₆-cycloalkyle, C₃-C₆-cycloalkyl-C₁-C₄-alkyle, C₁-C₄-alcoxy-C₁-C₄-alkyle, C₁-C₄-alkylthio-C₁-C₄-alkyle ou phényl-C₁-C₄-alkyle), C₁-C₄-alkylsulfinyle, C₁-C₄-alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par C₁-C₄-alkyle ou C₁-C₄-halogénoalkyle), phényle (qui peut à son tour être substitué par halogène, cyano, nitro, C₁-C₄-alkyle ou C₁-C₄-halogénoalkyle), les radicaux hétéroaryle pyrrolyle, pyridyle, pyridyl-N-oxyde, pyrimidyle, imidazolyle, pyrazolyle,
oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, C₁-C₄-alkyle, C₁-C₄-halogénoalkyle, C₁-C₄-alcoxy, C₁-C₄-halogénoalcoxy, C₁-C₄-alcoxy-C₁-C₄-alkyle, C₁-C₄-alkylthio, C₁-C₄-alkylthio-C₁-C₄-alkyle et C₃-C₆-cycloalkyle), les radicaux hétéroarylalkyle triazolyl-C₁-C₄-alkyle, pyridyl-C₁-C₄-alkyle, pyrimidyl-C₁-C₄-alkyle ou oxadiazolyl-C₁-C₄-alkyle (qui peuvent à leur tour être substitués par C₁-C₄-alkyle) ou
phényle le cas échéant substitué par halogène, cyano, C₁-C₄-alkyle, C₁-C₄-halogénoalkyle, C₁-C₄-alcoxy, C₁-C₄-halogénoalcoxy, dioxolanyle, pipéridinonyle, pyrrolidinonyle ou dihydrodioxazinyle
ainsi que de sels et de N-oxydes des composés de formule (IB-1).

4. Utilisation de composés de formule (IB-1) selon la revendication 1, dans laquelle
G¹ représente N ou C-halogène,
G³ représente pyridyle le cas échéant substitué par pyrrolyle ou pyrimidinyle,
ainsi que de sels et de N-oxydes des composés de formule (IB-1).

5. Composés de formule (IB-1), 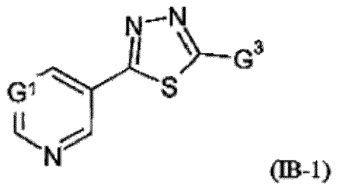 dans laquelle
G¹ représente N ou C-halogène,
G³ représente oxazolinyle, dihydrooxadiazinyle, dihydrodioxazinyle ou hydroxypyridyle, à chaque fois le cas échéant substitué par halogène, cyano, nitro, alkyle, halogénoalkyle, cycloalkyle, alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylcarbonylamino, alcoxycarbonylamino, alcoxyalkyle, halogénoalcoxyalkyle, alcényle, alcynyle, alkylcycloalkyle, alkylcarbonyle, alcoxycarbonyle, aminocarbonyle, pyridyle ou pyrimidyle,
hétaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle, le cas échéant substitué par halogène, nitro, amino, alkylamino, dialkylamino, alkyle, halogénoalkyle, cycloalkylalkyle, alcoxy, halogénoalcoxy, alkylthio, alcoxyalkyle, bis(alcoxy)alkyle, alcoxycarbonyle, alpha-hydroxyiminoalcoxycarbonylméthyle, alpha-alcoxyiminoalcoxycarbonylméthyle, C(X)NR²R³, (où X représente oxygène ou soufre, R² représente hydrogène ou alkyle et R³ représente alkyle, halogénoalkyle, alcoxy, cyanoalkyle, alcynyle, cycloalkyle, cycloalkylalkyle, alcoxyalkyle, alkylthioalkyle ou arylalkyle), alkylthio, alkylsulfinyle, alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par alkyle ou halogénoalkyle), phényle (qui peut à son tour être substitué par halogène, cyano, nitro, alkyle ou halogénoalkyle), les radicaux hétéroaryle pyrrolyle, pyridyle, pyridyl-N-oxyde, pyrimidyle, imidazolyle, pyrazolyle, oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, alkyle, halogénoalkyle, alcoxy, halogénoalcoxy, alcoxyalkyle, alkylthio, alkylthioalkyle et cycloalkyle), les radicaux hétéroarylalkyle triazolylalkyle, pyridylalkyle, pyrimidylalkyle ou oxadiazolylalkyle (qui peuvent à leur tour être substitués par alkyle) ou
phényle le cas échéant substitué par halogène, cyano, alkyle, halogénoalkyle, alcoxy, halogénoalcoxy, dioxolanyle, pipéridinonyle, pyrrolidinonyle ou dihydrodioxazinyle.

6. Composés de formule (IB-1) selon la revendication 5, dans laquelle
G¹ représente N ou C-halogène,
G³ représente à chaque fois oxazolinyle, dihydrooxadiazinyle, dihydrodioxazinyle ou hydroxypyridyle, à chaque fois le cas échéant substitué par halogène, cyano, nitro, C₁-C₆-alkyle, C₁-C₆-halogénoalkyle, C₃-C₆-cycloalkyle, C₁-C₆-alcoxy, C₁-C₆-halogénoalcoxy, C₁-C₆-alkylthio, C₁-C₆-halogénoalkylthio, C₁-C₆-alkylsulfinyle, C₁-C₆-alkylsulfonyle, C₁-C₆-halogénoalkylsulfinyle, C₁-C₆-halogénoalkylsulfonyle, amino, C₁-C₆-alkylamino, di-(C₁-C₆-alkyl)amino, C₁-C₆-alkylcarbonylamino, C₁-C₆-alcoxycarbonylamino, C₁-C₆-alcoxy-C₁-C₆-alkyle, C₁-C₆-halogénoalcoxy-C₁-C₆-alkyle, C₂-C₆-alcényle, C₂-C₆-alcynyle, C₁-C₆-alkyl-C₃-C₆-cycloalkyle, C₁-C₆-alkylcarbonyle, C₁-C₆-alcoxycarbonyle, aminocarbonyle, pyridyle ou pyrimidyle,
pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle, à chaque fois le cas échéant substitué par halogène, nitro, amino, C₁-C₆-alkylamino, di-(C₁-C₆-alkyl)amino, C₁-C₆-alkyle, C₁-C₆-halogénoalkyle, C₃-C₆-cycloalkyl-C₁-C₆-alkyle, C₁-C₆-alcoxy, C₁-C₆-halogénoalcoxy, C₁-C₆-alkylthio, C₁-C₆-alcoxy-C₁-C₆-alkyle, bis (C₁-C₆-alcoxy) -C₁-C₆-alkyle, C₁-C₆-alcoxycarbonyle, alpha-hydroxyimino-C₁-C₆-alcoxycarbonylméthyle, alpha-C₁-C₆-alcoxyimino-C₁-C₆-alcoxycarbonylméthyle, C(X)NR²R³, (où X représente oxygène ou soufre, R² représente hydrogène ou C₁-C₆-alkyle et R³ représente C₁-C₆-alkyle, C₁-C₆-halogénoalkyle, C₁-C₆-alcoxy, cyano-C₁-C₆-alkyle, C₂-C₆-alcynyle, C₃-C₆-cycloalkyle, C₃-C₆-cycloalkyl-C₁-C₆-alkyle, C₁-C₆-alcoxy-C₁-C₆-alkyle, C₁-C₆-alkylthio-C₁-C₆-alkyle ou phényl-C₁-C₆-alkyle), C₁-C₆-alkylsulfinyle, C₁-C₆-alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par C₁-C₆-alkyle ou C₁-C₆-halogénoalkyle), phényle (qui peut à son tour être substitué par halogène, cyano, nitro, C₁-C₆-alkyle ou C₁-C₆-halogénoalkyle), les radicaux hétéroaryle pyrrolyle, pyridyle, pyridyl-N-oxyde, pyrimidyle, imidazolyle, pyrazolyle, oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, C₁-C₆-alkyle, C₁-C₆-halogénoalkyle, C₁-C₆-alcoxy, C₁-C₆-halogénoalcoxy, C₁-C₆-alcoxy-C₁-C₆-alkyle, C₁-C₆-alkylthio, C₁-C₆-alkylthio-C₁-C₆-alkyle et C₃-C₆-cycloalkyle), les radicaux hétéroarylalkyle triazolyl-C₁-C₆-alkyle, pyridyl-C₁-C₆-alkyle, pyrimidyl-C₁-C₆-alkyle ou oxadiazolyl-C₁-C₆-alkyle (qui peuvent à leur tour être substitués par C₁-C₆-alkyle) ou
phényle le cas échéant substitué par halogène, cyano, C₁-C₆-alkyle, C₁-C₆-halogénoalkyle, C₁-C₆-alcoxy, C₁-C₆-halogénoalcoxy, dioxolanyle, pipéridinonyle, pyrrolidinonyle ou dihydrodioxazinyle.

7. Composés de formule (IB-1) selon la revendication 5, dans laquelle
G¹ représente N ou C-halogène,
G³ représente à chaque fois oxazolinyle, dihydrooxadiazinyle, dihydrodioxazinyle ou hydroxypyridyle, le cas échéant substitué par halogène, cyano, nitro, C₁-C₄-alkyle, C₁-C₄-halogénoalkyle, C₃-C₆-cycloalkyle, C₁-C₄-alcoxy, C₁-C₄-halogénoalcoxy, C₁-C₄-alkylthio, C₁-C₄-halogénoalkylthio, C₁-C₄-alkylsulfinyle, C₁-C₄-alkylsulfonyle, C₁-C₄-halogénoalkylsulfinyle, C₁-C₄-halogénoalkylsulfonyle, amino, C₁-C₄-alkylamino, di-(C₁-C₄-alkyl)amino, C₁-C₄-alkylcarbonylamino, C₁-C₄-alcoxycarbonylamino, C₁-C₄-alcoxy-C₁-C₄-alkyle, C₁-C₄-halogénoalcoxy-C₁-C₄-alkyle, C₂-C₄-alcényle, C₂-C₄-alcynyle, C₁-C₄-alkyl-C₃-C₆-cycloalkyle, C₁-C₄-alkylcarbonyle, C₁-C₄-alcoxycarbonyle, aminocarbonyle, pyridyle ou pyrimidyle
pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzo-imidazolyle, 2,1,3-benzoxadiazole, quinoléinyle, isoquinoléinyle, cinnolinyle, phtalazinyle, quinazolinyle, quinoxalinyle, naphtyridinyle, benzotriazinyle, purinyle, ptéridinyle et indolizinyle à chaque fois le cas échéant substitué par halogène, nitro, amino, C₁-C₄-alkylamino, di-(C₁-C₄-alkyl)amino, C₁-C₄-alkyle, C₁-C₄-halogénoalkyle, C₃-C₆-cycloalkyl-C₁-C₄-alkyle, C₁-C₄-alcoxy, C₁-C₄-halogénoalcoxy, C₁-C₄-alkylthio, C₁-C₄-alcoxy-C₁-C₄-alkyle, bis (C₁-C₄-alcoxy) -C₁-C₄-alkyle, C₁-C₄-alcoxycarbonyle, alpha-hydroxyimino-C₁-C₄-alcoxycarbonylméthyle, alpha-C₁-C₄-alcoxyimino-C₁-C₄-alcoxycarbonylméthyle, C(X)NR²R³, (où X représente oxygène ou soufre, R² représente hydrogène ou C₁-C₄-alkyle et R³ représente C₁-C₄-alkyle, C₁-C₄-halogénoalkyle, C₁-C₄-alcoxy, cyano-C₁-C₄-alkyle, C₂-C₄-alcynyle, C₃-C₆-cycloalkyle, C₃-C₆-cycloalkyl-C₁-C₄-alkyle, C₁-C₄-alcoxy-C₁-C₄-alkyle, C₁-C₄-alkylthio-C₁-C₄-alkyle ou phényl-C₁-C₄-alkyle), C₁-C₄-alkylsulfinyle, C₁-C₄-alkylsulfonyle, les radicaux hétérocyclyle morpholinyle, triazolinonyle, dihydrodioxazinyle, dihydrooxadiazinyle, dioxolanyle, dioxanyle, pipéridinonyle, pyrrolidinonyle et pyrazolinonyle (qui peuvent à leur tour être substitués par C₁-C₄-alkyle ou C₁-C₄-halogénoalkyle), phényle (qui peut à son tour être substitué par halogène, cyano, nitro, C₁-C₄-alkyle ou C₁-C₄-halogénoalkyle), les radicaux hétéroaryle pyrrolyle, pyridyle, pyridyl-N-oxyde, pyrimidyle, imidazolyle, pyrazolyle, oxazolyle, thiazolyle, furannyle, thiényle, triazolyle, oxadiazolyle, thiadiazolyle, pyrazinyle, triazinyle, tétrazinyle et isoquinoléinyle (qui peuvent à leur tour être substitués par halogène, nitro, C₁-C₄-alkyle, C₁-C₄-halogénoalkyle, C₁-C₄-alcoxy, C₁-C₄-halogénoalcoxy, C₁-C₄-alcoxy-C₁-C₄-alkyle, C₁-C₄-alkylthio, C₁-C₄-alkylthio-C₁-C₄-alkyle et C₃-C₆-cycloalkyle), les radicaux hétéroarylalkyle triazolyl-C₁-C₄-alkyle, pyridyl-C₁-C₄-alkyle, pyrimidyl-C₁-C₄-alkyle ou oxadiazolyl-C₁-C₄-alkyle (qui peuvent à leur tour être substitués par C₁-C₄-alkyle) ou
phényle le cas échéant substitué par halogène, cyano, C₁-C₄-alkyle, C₁-C₄-halogénoalkyle, C₁-C₄-alcoxy, C₁-C₄-halogénoalcoxy, dioxolanyle, pipéridinonyle, pyrrolidinonyle ou dihydrodioxazinyle.

8. Composés de formule (IB-1) selon la revendication 5, dans laquelle G¹ représente N ou C-halogène et
G³ représente pyridyle le cas échéant substitué par pyrimidinyle.

9. Agent, **caractérisé par** une teneur en au moins un composé de formule (IB-1) selon les revendications 5 à 8.

10. Procédé non-thérapeutique pour lutter contre les organismes nuisibles, **caractérisé en ce qu'**on laisse agir un composé de formule (IB-1) selon les revendications 5 à 8 ou un agent selon la revendication 9 sur les organismes nuisibles et/ou leur espace de vie.
